(19) Europäisches Patentamt
European Patent Office
Office européen des brevets

(11) Veröffentlichungsnummer : **0 318 425 B1**

(12) # EUROPÄISCHE PATENTSCHRIFT

(45) Veröffentlichungstag der Patentschrift : **18.03.92 Patentblatt 92/12**

(21) Anmeldenummer : **88730257.8**

(22) Anmeldetag : **25.11.88**

(51) Int. Cl.$^5$ : **C07C 69/635,** C07C 67/14, C07C 53/23, C07C 53/50, C07C 67/08, A01N 53/00, C07C 233/01, C07C 271/06

(54) **2,2-Difluorcyclopropylethanderivate, Verfahren zu ihrer Herstellung und ihre Verwendung als Schädlingsbekämpfungsmittel.**

(30) Priorität : **27.11.87 DE 3740840**

(43) Veröffentlichungstag der Anmeldung : **31.05.89 Patentblatt 89/22**

(45) Bekanntmachung des Hinweises auf die Patenterteilung : **18.03.92 Patentblatt 92/12**

(84) Benannte Vertragsstaaten : **AT BE CH DE ES FR GB GR IT LI LU NL SE**

(56) Entgegenhaltungen : **DE-A- 3 504 749**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Wegner, Peter, Dr.**
**Am Eichenhain 60**
**W-1000 Berlin 28 (DE)**
Erfinder : **Joppien, Hartmut, Dr.**
**Juttastrasse 18**
**W-1000 Berlin 37 (DE)**
Erfinder : **Hömberger, Günter, Dr.**
**Waldmannstrasse 21**
**W-1000 Berlin 46 (DE)**
Erfinder : **Köhn, Arnim, Dr.**
**Alt-Wittenau 63c**
**W-1000 Berlin 26 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft neue 2,2-Difluorcyclopropylethanderivate, ihre Herstellung sowie Zwischenprodukte zu ihrer Herstellung und ihre Verwendung als Schädlingsbekämpfungsmittel, insbesondere gegen Insekten und Milben.

Es ist bereits bekannt, daß Cyclopropanverbindungen akarizide Eigenschaften besitzen (USP 3.995.054).

Nachteilig bei den bekannten Verbindungen ist jedoch, daß sie nicht ausreichend insektizid und akarizid wirksam sind.

Die Aufgabe der vorliegenden Erfindung lag darin, Verbindungen zur Verfügung zu stellen, welche Insekten und Milben besser als die für diesen Zweck bekannten Verbindungen bekämpfen.

Es wurde nun gefunden, daß 2,2-Difluorcyclopropylethanderivate der allgemeinen Formel I

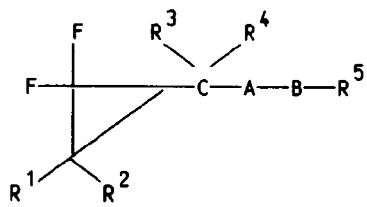

(I),

in der

$R^{1-4}$ gleich oder verschieden sein können und Wasserstoff, $C_{1-6}$-Alkyl und/oder Halogen darstellen und

$R^5$ Wasserstoff,

ein Alkalimetallatom oder ein entsprechendes Äquivalent eines zweiwertigen Metalles,

$C_{1-20}$-Alkyl, $C_{2-20}$-Alkenyl, $C_{2-20}$-Alkinyl,

Halogen-$C_{1-10}$-alkyl,

$C_{3-6}$-Cycloalkyl,

$C_{1-3}$-Alkyl-$C_{3-6}$-cycloalkyl,

$C_{3-6}$-Cycloalkyl-$C_{1-6}$-alkyl,

Halogen-$C_{3-6}$-cycloalkyl-$C_{1-6}$-alkyl,

Decalinyl,

Indanyl,

Adamantyl, Adamantylmethyl,

Difluorcyclopropylethylcarbonyloxy-$C_{1-10}$-alkyl,

Difluorcyclopropylethylcarbonyloxydecalinyl,

Difluorcyclopropylcarbonyloxy-$C_{1-3}$-alkoxy-$C_{1-3}$-alkyl,

Phenyl-$C_{1-6}$-alkyl,

Phenyl-$C_{2-6}$-alkenyl,

Halogenbenzyl,

$C_{1-4}$-Alkylbenzyl,

$C_{1-3}$-Alkoxyphenyl-$C_{1-6}$-alkyl,

Phenoxybenzyl,

α-Cyanophenoxybenzyl,

α-$C_{1-3}$-Alkylphenoxybenzyl,

Ethoxy-(α-trifluormethyl)benzyl,

Halogenphenyl(cyclopropyl)-$C_{1-3}$-alkyl,

Halogenphenoxy-$C_{1-6}$-alkyl,

Naphthyl-$C_{1-6}$-alkyl,

Methylthiazolyl-$C_{1-6}$-alkyl,

Tris(difluorcyclopropylmethylcarbonyloxymethyl)methyl,

Tri($C_{4-8}$-cycloalkyl)stannyl, freies Aryl oder durch $C_{1-20}$-Alkyl, Halogen-$C_{1-6}$-alkyl, $C_{1-16}$-Alkoxy, Halogen-$C_{1-6}$-alkoxy, Phenyl-$C_{1-6}$-alkyl, Phenyl-$C_{1-6}$alkoxy, $C_{3-10}$-Cycloalkyloxy, Halogen$C_{3-10}$-cycloalkyloxy, $C_{3-6}$-Cycloalkylalkoxy, Halogen-$C_{3-6}$-cycloalkylalkoxy, $C_{2-6}$-Alkenyloxy, Halogen-$C_{2-6}$alkenyloxy, $C_{2-6}$-Alkinyloxy, Halogen-$C_{2-6}$-alkinyloxy, Alkylsulfonyloxy, Alkylphenylsulfonyloxy, Halogenalkylsulfonyloxy, Phenyl, Halogen, Amino, Cyano, Hydroxy, Nitro, Aryloxy, Hetereoaryloxy, Halogenaryloxy, Arylamino, Halogenarylamino, $C_{1-6}$-

Alkoxycarbonyl, $C_{1-6}$-Alkoxycarbonylmethyl, Halogen-$C_{1-6}$alkoxycarbonyl, $C_{1-2}$-Alkyldioxy, $C_{1-6}$-Alkylthio, Halogen-$C_{3-6}$-cycloalkylalkylamino, Halogen-$C_{3-6}$-cycloalkylalkylcarbonyloxy, $C_{1-6}$-Alkylamino, oder Di-$C_{1-6}$-alkylamino ein- odermehrfach substituiertes Aryl, freies Heteroaryl, durch Halogen, $C_{1-3}$-Alkyl oder Halogen-$C_{1-3}$-alkyl substituiertes Heteroaryl sowie die Gruppe $CONHR^8$ bedeutet, wobei

$R^8$ Wasserstoff, $C_{1-6}$-Alkyl, Phenyl oder durch $C_{1-6}$-Alkyl, Halogen-$C_{1-6}$-alkyl, $C_{1-16}$-Alkoxy, Halogen-$C_{1-6}$-alkoxy, Phenyl-$C_{1-6}$-alkoxy, $C_{3-10}$-Cycloalkyloxy, Halogen-$C_{3-10}$-cycloalkyloxy, $C_{3-6}$-Cycloalkylalkoxy, Halogen-$C_{3-6}$-cycloalkylalkoxy, $C_{2-6}$-Alkenyloxy, Halogen-$C_{2-6}$-alkenyloxy, $C_{2-6}$-Alkinyloxy, Halogen-$C_{2-6}$-alkinyloxy, Alkylsulfonyloxy, Halogenalkylsulfonyloxy, Phenyl, Halogen, Amino, Cyano, Hydroxy, Nitro, Aryloxy, Halogenaryloxy, Arylamino, Halogenarylamino, $C_{1-6}$-Alkoxycarbonyl, Halogen-$C_{1-6}$-alkoxycarbonyl, $C_{1-2}$-Alkyldioxy, $C_{1-6}$-Alkylthio, Halogen-$C_{3-6}$-cycloalkylalkylamino, $C_{1-6}$-Alkylamino, oder Di-$C_{1-6}$-alkylamino ein- oder mehrfach substituiertes Phenyl bedeutet,

A für eine Carbonyl-, Thiocarbonyl oder Methylen-Gruppe und

B für Sauerstoff, Schwefel oder die Gruppe $NR^6$ steht, in der

$R^6$ Wasserstoff oder $C_{1-6}$-Alkyl, $C_{3-12}$-Cycloalkyl, Phenyl sowie die Gruppe $CO\text{-}R^7$ bedeutet, wobei

$R^7$ $C_{1-6}$-Alkyl, Halogen-$C_{1-6}$-alkyl, Phenyl oder durch $C_{1-6}$-Alkyl, halogen-$C_{1-6}$-alkyl, $C_{1-16}$-Alkoxy, Halogen-$C_{1-6}$-alkoxy, Phenyl-$C_{1-6}$-alkoxy, $C_{3-10}$-Cycloalkyloxy, Halogen-$C_{3-10}$-cycloalkyloxy, $C_{3-6}$-Cycloalkylalkoxy, Halogen-$C_{3-6}$-cycloalkylalkoxy, $C_{2-6}$-Alkenyloxy, Halogen-$C_{2-6}$-alkenyloxy, $C_{2-6}$-Alkinyloxy, Halogen-$C_{2-6}$-alkinyloxy, Alkylsulfonyloxy, Halogenalkylsulfonyloxy, Phenyl, Halogen, Amino, Cyano, Hydroxy, Nitro, Aryloxy, Halogenaryloxy, Arylamino, Halogenarylamino, $C_{1-6}$-Alkoxycarbonyl, Halogen-$C_{1-6}$-alkoxycarbonyl, $C_{1-2}$-Alkyldioxy, $C_{1-6}$-Alkylthio, Halogen-$C_{3-6}$-cycloalkylalkylamino, $C_{1-6}$-Alkylamino, oder Di-$C_{1-6}$alkylamino ein- oder mehrfach substituiertes Phenyl, $C_{3-6}$-Cycloalkyl, $C_{1-6}$ Alkylamino oder $C_{1-6}$-Alkoxy bedeutet.

eine im Vergleich zu den bekannten Verbindungen verbesserte insektizide und akarizide Wirksamkeit zeigen.

Der Begriff "Alkyl" steht für eine geradlinige oder verzweigte Kette von Kohlenstoffatomen.

Der Begriff "Alkenyl" steht für eine geradlinige oder verzweigte Kette von Kohlenstoffatomen, die durch Doppelbindungen ein- oder mehrfach unterbrochen ist.

Der Begriff "Alkinyl" steht für eine geradlinige oder verzweigte Kette von Kohlenstoffatomen, die durch Dreifachbindungen ein- oder mehrfach unterbrochen ist.

Der Begriff "Aryl" steht für einen ein- bis dreikernigen aromatischen Rest, wie z.B. Phenyl, Naphthyl oder Phenanthryl.

Der Begriff "Heteroaryl" steht für einen 5- oder 6-gliedrigen Ring, der ein oder mehrere Stickstoff-, Sauerstoff- und/oder Schwefelatome enthält, gesättigt, teilgesättigt oder ungesättigt sein kann und gegebenenfalls benzoannelliert ist, wie z.B. Pyridin, Thiazol oder Chromen.

$R^5$ und $R^6$ können gegebenenfalls gemeinsam mit dem N-Atom gesättigte oder ungesättigte heterocyclishe Ringe darstellen, wie z.B. Morpholino, Piperidino, Pyrrolo, Imidazolo oder Triazolo.

Die Verbindungen der allgemeinen Formel I liegen als racemische Gemische der optisch aktiven Isomere vor. Die Erfindung betrifft somit nicht nur die Isomerengemische, sondern auch jedes einzelne Isomer der erfindungsgemäßen Verbindungen.

Als besonders wirksam haben sich solche 2,2-Difluorcyclopropylethanderivate der Formel I erwiesen, bei denen

A eine Carbonylgruppe,

B Sauerstoff oder Gruppe NH,

$R^{1-4}$ Wasserstoff und

$R^5$ Wasserstoff, $C_{1-20}$-Alkyl, $C_{2-20}$-Alkenyl, $C_{2-20}$-Alkinyl, m-Phenoxy-benzyl und Naphthylmethyl bedeuten.

Die Erfindung betrifft des weiteren Verbindungen der allgemeinen Formel II

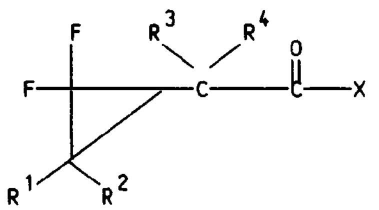

(II),

worin

$R^{1-4}$ die in Formel I angegebene Bedeutung haben und

X für OH, Cl oder Br steht,

als Zwischenprodukte zur Herstellung von Verbindungen der Formel I.

Die neuen Zwischenprodukte sind insbesondere vorteilhaft geeignet, um zu den erfindungsgemäßen Verbindungen der Formel I zu gelangen, bei denen A für eine Carbonylgruppe steht.

Die erfindungsgemäßen 2,2-Difluorcyclopropylethanderivate der Formel I, bei denen

A für eine Carbonylgruppe steht,

lassen sich herstellen, indem man entweder

A) ein Säurehalogenid der allgemeinen Formel II

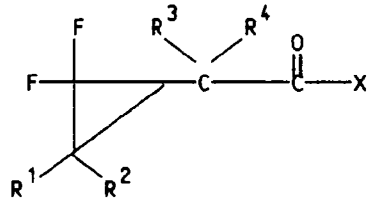

(II),

worin

$R^{1-4}$ die in Formel I angegebene Bedeutung haben und

X für Chlor oder Brom steht,

mit einem Alkohol oder Amin der allgemeinen Formel III

$$H\text{-}B\text{-}R^5 \qquad (III),$$

worin

B und $R^5$ die in Formel I angegebene Bedeutung haben, gegebenenfalls in einem Lösungsmittel in Gegenwart eines Säureacceptors umsetzt,

oder

B) eine freie Säure der allgemeinen Formel IV

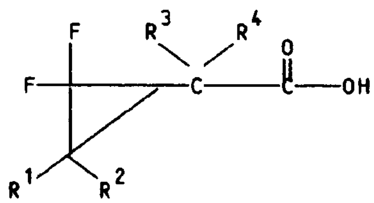

(IV),

worin

$R^{1-4}$ die in Formel I angegebene Bedeutung haben, mit einem Alkohol oder Amin der Formel III, gegebenenfalls unter Verwendung eines Lösungsmittels in Gegenwart eines Katalysators, umsetzt, oder

C) eine Säure der allgemeinen Formel V

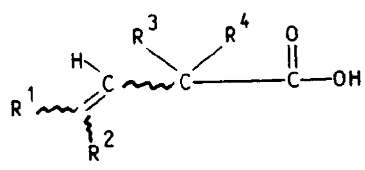

(V),

worin

$R^{1-4}$ die in Formel I angegebene Bedeutung haben, mit einem Alkohol oder Amin der Formel III, gegebenenfalls in einem Lösungsmittel in Gegenwart Katalysators oder wasserentziehender Mittel zu einer Zwischenverbindung der Formel VI

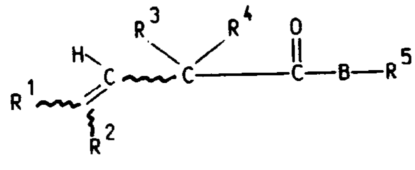

(VI),

worin

B und $R^{1-5}$ die in Formel I angegebene Bedeutung haben, umsetzt und diese in Gegenwart eines inerten Lösungsmittels mit Difluorcarben reagieren läßt, oder falls

A eine Methylengruppe bedeutet und

B die Gruppe $NR^6$ ist,

D) ein Amid der speziellen Formel I

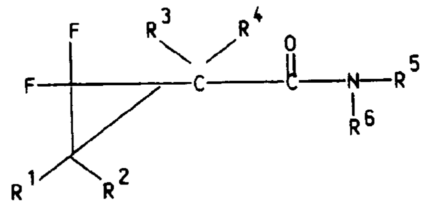

(I),

worin

$R^{1-6}$ die in Formel I angegebene Bedeutung haben, gegebenenfalls in einem Lösungsmittel in Gegenwart eines Katalysators, mit einem Reduktionsmittel umsetzt, oder

E) ein Amin der allgemeinen Formel VII

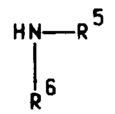

(VII),

worin

$R^5$ und $R^6$ die in Formel I angegebene Bedeutung haben, in Gegenwart einer Base, mit einem Halogenid der Formel VIII

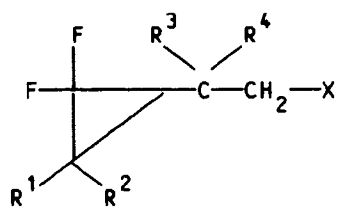

(VIII),

worin

$R^{1-4}$ die in Formel I angegebene Bedeutung haben und X für Chlor oder Brom steht, gegebenenfalls unter Verwendung eines Lösungsmittels, umsetzt, oder

F) Verbindungen der allgemeinen Formel IX

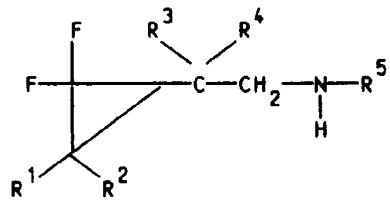

(IX),

worin
$R^{1-5}$ die in Formel I angegebene Bedeutung haben und nach der Reaktionsvariante D) oder E) hergestellt werden, mit Säurehalogeniden der allgemeinen Formel X

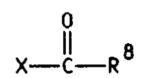

(X),

worin
$R^8$ die in Formel I angegebene Bedeutung hat und X für Chlor oder Brom steht, gegebenenfalls unter Verwendung eines Lösungsmittels in Gegenwart eines Säureacceptors, umsetzt, oder falls
B Sauerstoff ist,
G) einen Alkohol der allgemeinen Formel XI

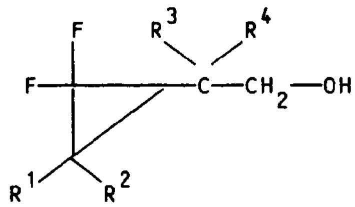

(XI),

worin
$R^{1-4}$ die in Formel I angegebene Bedeutung haben, mit einem Isocyanat der allgemeinen Formel XII

$$O{=}C{=}N\text{-}R^8 \qquad \text{(XII)},$$

worin
$R^8$ die in Formel I angegebene Bedeutung hat, gegebenenfalls unter Verwendung eines Lösungsmittels in Gegenwart eines Katalysators, umsetzt, und gegebenenfalls anschließend die so erhaltenen racemischen Gemische in die optisch aktiven Isomeren nach an sich bekannten Methoden auftrennt.

Als Säureacceptor für die Durchführung der Reaktionsvarianten A) und F) eignen sich die üblichen basischen Mittel, insbesondere aliphatische, aromatische und heterocyclische Amine, wie z. B. Triethylamin, Dimethylamin, Piperidin, Dimethylanilin, Dimethylbenzylamin, Pyridin und Dimethylaminopyridin oder anorganische Basen wie Oxide, Hydroxide, Carbonate, Hydrogencarbonate und Alkoholate von Alkali- und Erdalkalimetallen wie Kaliumhydroxid, Natriumhydroxid, Natrium- und Kaliumcarbonat.

Als Lösungsmittel eignen sich die vorgenannten Säureacceptoren selbst oder inerte Lösungsmittel oder Gemische derselben untereinander.

Genannt seien beispielsweise aliphatische, alicyclische und aromatische Kohlenwasserstoffe, die gegebenenfalls chloriert sein können wie Hexan, Cyclohexan, Petrolether, Benzol, Toluol, Xylol, Methylenchlorid, Chloroform, Kohlenstofftetrachlorid, Ethylenchlorid, Trichlorethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Benzonitril; Ester wie Ethylacetat und Amylacetat; Säureamide wie Dimethylformamid und Dimethylacetamid sowie Sulfone und Sulfoxide wie Dimethylsulfoxid und Sulfolan.

Die Umsetzung kann innerhalb eines weiten Temperaturbereiches durchgeführt werden. Im allgemeinen wird sie bei einer Temperatur zwischen -20°C und dem Siedepunkt der Reaktionsmischung, vorzugsweise zwischen 20°C und 200°C, durchgeführt.

Die Umsetzung wird unter dem Druck der Umgebung durchgeführt, wenngleich sie auch bei erhöhtem oder vermindertem Druck durchgeführt werden könnte.

Als Katalysatoren für die Durchführung der Reaktionsveriante B) eignen sich starke Säuren wie Schwefelsäure, Halogenwasserstoff, Sulfonsäuren und saure Ionenaustauscher. Es ist vorteilhaft, dem Reaktionsgemisch das Wasser oder den Ester der Formel I zu entziehen, z. B. durch azeotrope Destillation oder durch Bindung des Wassers an Schwefel- oder eine Halogenwasserstoffsäure.

Die Reaktionsvariante B) kann unter den gleichen Reaktionsbedingungen in bezug auf Temperatur und Druck und in den gleichen Lösungsmitteln oder Gemischen derselben durchgeführt werden wie sie für die Reaktionsvariante A) genannt wurden.

Für die Durchführung der Reaktionsvariante C) eignen sich zur Herstellung der Zwischenverbindung VI die gleichen sauren Katalysatoren und inerten Lösungsmittel wie sie für Reaktionsvariante B) genannt wurden. Besonders geeignet für die Veresterung ist die Bindung des Wassers durch die Kombination von Triphenylphosphin und Azodicarbonsäureester (Synthesis 1981, 1). Geeignet sind aber auch die klassischen wasserentziehenden Mittel wie konzentrierte Schwefelsäure, wasserfreie Salze von anorganischen Säuren wie Magnesiumsulfat oder Calciumchlorid, Carbodiimide wie das Dicyclohexylcarbodiimid oder auch Zeolithe. Die Carbenreaktion wird jedoch bevorzugt in Ethern wie Diglyme, Triglyme und Tetraglyme durchgeführt. Die Erzeugung von Difluorcarben erfolgt nach Fachliteratur bekannten Methoden (Burton und Hahnfeld, Fluorine Chem. Rev. 8 (1977), 119 ff.).

Als Difluorcarbenspender eignen sich beispielsweise Alkalimetallchlordifluoracetate wie Natriumchloridfluoracetat; Halogendifluorkohlenwasserstoffe wie Chlordifluormethan; Organozinn-Verbindungen wie Trimethyl(trifluormethyl)-zinn; Organoquecksilber-Verbindungen wie Phenyl(trifluormethyl)quecksilber; Organophosphor Verbindungen wie Tris(trifluormethyl)-difluorphosphoran und Triphenyl(bromidfluormethyl)-phosphoniumbromid.

Die Reaktionsvariante D) kann unter den gleichen Reaktionsbedingungen in bezug auf Temperatur und Druck und in den gleichen Lösungsmitteln oder Gemischen derselben durchgeführt werden, wie sie für die Reaktionsvariante A) genannt wurden.

Als Reaktionsmittel für die Durchführung der Reaktionsvariante D) eignet sich z. B. Lithiumaluminiumhydrid, Boran oder Natriumborhydrid in Gegenwart von Kobaltdichlorid, Essigsäure oder Trifluoressigsäure.

Die Reaktionsvariante E) kann unter den gleichen Reaktionsbedingungen in bezug auf Temperatur und Druck und in den gleichen Lösungsmitteln oder Gemischen derselben durchgeführt werden, wie sie für die Reaktionsvariante A) genannt wurden.

Als Base für die Durchführung der Reaktionsvariante E) eignet sich z. B. Kaliumcarbonat, Kaliumhydroxid, Natriumhydrid oder Kalium-tert.-butylat.

Die Reaktionsvariante G) kann unter den gleichen Reaktionsbedinungen in bezug auf Temperatur und Druck und in den gleichen Lösungsmitteln oder Gemischen derselben durchgeführt werden, wie sie für die Reaktionsvariante A) genannt wurden.

Die Herstellung der optischen Isomeren der erfindungsgemäßen Verbindungen kann nach an sich bekannten Verfahren durchgeführt werden, beispielsweise durch Umsetzen von Verbindungen der allgemeinen Formel II mit einem chiralen Hilfsreagenz, wie z.B. einem optisch aktiven Amin oder einem optisch aktiven Alkohol und anschließender Trennung der so erhaltenen Diastereomeren mit Hilfe physikalischer Methoden (Tetrahedron 33, 2725 (1977)), wie z.B. Kristallisation, Destillation oder Fest-Flüssig-Chromatographie. Durch eine anschließende hydrolytische Spaltung, die entweder säure- oder basenkatalytisch geführt werden kann, erhält man die optischen Isomeren der freien Säuren der allgemeinen Formel IV, die nach Verfahrensvariante B) zu den erfindungsgemäßen Verbindungen umgesetzt werden können.

Weiterhin können die bei der Synthese entstehenden Gemische optischer Isomere der allgemeinen Formel I durch Chromatographie an chiralen stationären Phasen, wie z.B. an Cyclodextrinen, Stärke oder an Polymere gebundene optisch aktive Aminosäurederivate, in die Enantiomere getrennt werden (Angew. Chem. 92, 14 (1980)).

Die nach oben genannten Verfahren herstellbaren erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb- und geruchslose Flüssigkeiten sowie Kristalle dar, die schwerlöslich in Wasser, bedingt löslich in aliphatischen Kohlenwasserstoffen wie Petrolether, Hexan, Pentan und Cyclohexan, gut löslich in halogenierten Kohlenwasserstoffen wie Chloroform, Methylenchlorid und Tetrachlorkohlenstoff, aromatischen Kohlenwasserstoffen wie Benzol, Toluol und Xylol, Ethern wie Diethylether, Tetrahydrofuran und Dioxan, Carbonsäurenitrilen wie Acetonitril, Alkoholen wie Methanol und Ethanol, Carbonsäureamiden wie Dimethylformamid und Sulfoxiden wie Dimethylsulfoxid, sind.

Das als Ausgangsmaterial zu verwendende Säurehalogenid der Formel X, die Säure der Formel V, der Alkohol und das Amin der Formel III und VII sowie das Isocyanat der Formel XII sind bekannt oder können nach an sich bekannten Methoden hergestellt werden.

Das Säurehalogenid der Formel II, das Halogenid der Formel VIII sowie der Alkohol der Formel XI können nach an sich bekannten Methoden hergestellt werden.

Die erfindungsgemäßen Verbindungen zeichnen sich durch gute insektizide Wirkung, insbesondere durch eine akarizide Wirkung aus und stellen damit eine wertvolle Bereicherung der Tecknik dar. Aufgrund ihrer Wirkung gegen eine weite Spanne saugender Arthropoden können die erfindungsgemäßen Verbindungen nicht nur gegen Schädlinge an Kulturpflanzen eingesetzt werden, sondern auch zur Bekämpfung von Parasiten des Menschen und der Nutztiere. Besondere Bedeutung hat die Wirkung der erfindungsgemäßen Verbindungen gegen Parasiten, die hinsichtlich anderer Mittel Resistenz entwickelt haben. Da die erfindungsgemäßen Verbindungen von den Pflanzen aufgenommen und im Gefäßsystem transportiert werden, können sie auch in den Boden appliziert werden und gelangen somit auch in solche Pflanzenteile, die kaum direkt getroffen werden können.

Zu den Insekten und Milben, einschließlich tierischer Ektoparasiten, die erfindungsgemäß bekämpft werden können, gehören beispielsweise die Lepidopteren wie Plutella xylostella, Spodoptera littoralis, Heliothis armigera und Pieris brassicae; die Dipteren wie Musca domestica, Ceratitis capitata, Erioischia brassicae, Lucilia sericata und Aedes aegypti; die Homopteren einschließlich Blattläusen wie Megoura viciae und Nilaparvata lugens; die Coleopteren wie Phaedon cochleariae, Anthonomus grandis und Cornrootworm (Diabrotica spp., z. B. Diabrotica undecimpunctata); die Othopteren wie Blattella germanica; die Zecken wie Boophilus microplus und die Läuse wie Damalinia bovis und Linognathus vituli sowie die Spinnmilben wie Tetranychus urticae und Panoychus ulmi.

Die Anwendung der erfindungsgemäßen Verbindungen kann in Konzentrationen von 0,0005 bis 5,0 %, vorzugsweise von 0,001 bis 0,1 % erfolgen, worunter das Gewicht in Gramm Wirkstoff in 100 ml Zubereitung zu verstehen ist.

Die erfindungsgemäßen Verbindungen können entweder allein, in Mischung miteinander oder mit anderen insektiziden Wirkstoffen angewendet werden. Gegebenenfalls können andere Pflanzenschutz- oder Schädlingsbekämpfungsmittel wie zum Beispiel Insektizide, Akarizide oder Fungizide, je nach dem gewünschten Zweck zugesetzt werden.

Eine Förderung der Wirkungsintensität und der Wirkungsgeschwindigkeit kann zum Beispiel durch wirkungssteigernde Zusätze wie organische Lösungsmittel, Netzmittel und Öle erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die erfindungsgemäßen Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Tonsil, Silicagel, Talkum, Kaolin, Attapulgit, Kalkstein und pflanzliche Produkte, zum Beispiel Mehle.

An oberflächenaktiven Stoffen sind zum Beispiele zu nennen Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und der Salze.

Zur Herstellung der Zubereitungen werden zum Beispiel die folgenden Bestandteile eingesetzt:

A SPRITZPULVER

20 Gew.-% Wirkstoff
35 Gew.-% Bleicherde
8 Gew.-% Calciumsalz der Ligninsulfonsäure
2 Gew.-% Natriumsalz des N-Methyl-N-oleyl-taurins
35 Gew.-% Kieselsäure

B PASTE

45 Gew.-% Wirkstoff
5 Gew.-% Natriumalunimiumsilikat
15 Gew.-% Cetylplyglycolether mit
8 Mol Ethylenoxid
2 Gew.-% Spindelöl
10 Gew.-% Polyethylenglykol
23 Teile Wasser

C EMULSIONSKONZENTRAT

20 Gew.-% Wirkstoff
75 Gew.-% Isophoron
5 Gew.-% einer Mischung auf Basis des Natriumsalzes des N-Methyl-N-oleyl-taurins und des Calciumsalzes der Ligninsulfonsäure.

Die folgenden Beispiele beschreiben die Herstellung der erfindungsgemäßen Verbindungen.

Beispiel 1

2-(2,2-Difluorcyclopropyl)-essigsäurebenzylester

80 g (454 mMol) 3-Butensäurebenzylester werden in 250 ml Diethylenglycoldimethylether (Diglyme) gelöst und bei 165°C innerhalb von 4 Stunden mit einer Lösung von 138 g (908 mMol) Natriumchloridfluoracetat, gelöst in 250 ml Diglyme, versetzt. Es wird eine Stunde bei 165°C nachgerührt und anschließend abgekühlt. Das ausgefallene Natriumchlorid wird abgesaugt und mit 100 ml Diglyme nachgewaschen. Im Vakuum (35°C, Ölpumpe) wird eingeengt und anschließend in 500 ml Ether aufgenommen, zweimal mit je 100 ml Wasser gewaschen und über Magnesiumsulfat getrocknet. Nach dem Eindampfen wird unter Ölpumpenvakuum über eine Dornenkolonne fraktioniert und eine Probe dünnschichtchromatographisch (Hexan: Essigester = 1 : 1) analysiert (RF = 0,64).

Ausbeute: 82,4 g (80 % d. Th.)
$Kp_{0,05}$: 90°C
$n_D^{20}$: 1,4783

Herstellung des Ausgangsmaterials

3-Butensäurebenzylester

Zu 46 g (535 mMol) 3-Butensäure, in 500 ml Diethylether gelöst, und 87 ml (535 mMol) Azodicarbonsäurediethylester tropft man 138 g (535 mMol) Triphenylphosphin und 84 ml (812 mMol) Benzylalkohol gelöst in 500 ml Diethylether und rührt 7 Stunden bei Raumtemperatur nach und läßt über Nacht stehen. Nach dem Verdampfen des Lösungmittels wird durch Säulenchromatographie (Krieselgel; Hexan : Essigester = 95 : 5) gereinigt und eine Probe dünnschichtchromatographisch (Hexan : Essigester = 1 : 1) analysiert (RF = 0,61).

Ausbeute: 83, 5 g (88 % d. Th.)
$n_D^{20}$: 1,5084

Beispiel 2

2,2-Difluorcyclopropylessigsäure

Zu einer Lösung von 35,2 g (630 mMol) Kaliumhydroxid in 360 ml Methanol tropft man unter Eiskühlung 142,3 g (630 mMol) 2-(2,2-Difluorcyclopropyl)-essigsäurebenzylester und rührt 3 Stunden bei Raumtemperatur (RT) nach. Anschließend engt man am Rotationsverdampfer ein, versetzt mit 500 ml Ether, saugt das ausgefallene Kaliumsalz ab und wäscht es dreimal mit Ether. Das Salz wird in 150 ml Wasser gelöst, die Lösung mit 54 ml konzentrierter Salzsäure angesäuert und dreimal mit je 200 ml Essigester extrahiert. Die Essigesterphasen werden zweimal mit Wasser gewaschen, werden über Magnesiumsulfat getrocknet und bei 40°C unter Normaldruck eingeengt. Anschließend wird unter Vakuum (17 mbar) fraktioniert und dünnschichtchromatographisch (Dichlormethan : Methanol = 95 : 5) analysiert (RF = 0,20).

Ausbeute: 82 g (96 % d. Th.)
$Kp_{17}$: 99 - 100°C

Beispiel 3

2-(2,2-Difluorcyclopropyl)-essigsäuretetradecylester

Eine Lösung von 2,08 g (9,7 mMol) 1-Tetradecanol in 20 ml Tetrahydrofuran (THF) wird mit 200 mg 4-Dimethylaminopyridin (DMAP) und 1,35 ml (9, 7 mMol) Triethylamin versetzt und auf 0 °C abgekühlt. Bei 0 - 5 °C wird eine Lösung von 1,5 g (9,7 mMol) 2-(2,2-Difluorcyclopropyl)-essigsäurechlorid in 10 ml THF zugetropft. Anschließend wird bei Raumtemperatur 2 Stunden nachgerührt. Man saugt das ausgefallene Triethylaminhydrochlorid ab, engt die Lösung unter vermindertem Druck ein und löst den Rückstand in 50 ml Diethylether. Nach Waschen mit je 10 ml Wasser, 10 % Natronlauge und Wasser bis zu neutralen Reaktion wird die Etherlösung über Magnesiumsulfat getrocknet und im Vakuum (200 mbar) eingedampft. Der Rückstand wird im Ölpumpenvakuum destilliert.

Ausbeute: 2,2 g (68 % d. Th.)
Kp (200 mbar): 110°C

Herstellung des Ausgangsmaterials

2-(2,2-Difluorcyclopropyl)-essigsäurechlorid

Eine Mischung aus 39,8 g (0,29 mMol) 2,2-Difluorcyclopropylessigsäure und 38,5 ml (0,53 mMol) Thionylchlorid wird 5 Stunden unter Rückfluß gekocht. Danach wird das überschüssige Thionylchlorid abdestilliert und der Rückstand im Vakuum (200 mbar) destilliert.

Ausbeute: 43 g (95 % d. Th.)
Kp (200 mbar): 94 °C

Beispiel 4

2-(2,2-Difluorcyclopropyl)-essigsäure-4-chloranilid

15,3 g (120 mMol) 4-Chloranilin, 18,4 ml (132 mMol) Triethylamin und 50 mg Dimethylaminopyridin, gelöst in 150 ml Tetrahydrofuran, werden unter Eiskühlung bei 35°C mit 18,6 g (120 mMol) 2-(2,2-Difluorcyclopropyl)-essigsäurechlorid versetzt. Es wird zwei Stunden bei Raumtemperatur nachgerührt und in 1000 ml Wasser eingegossen. Die ausgefallenen Kristalle werden abgesaugt, mit wenig verd. Salzsäure nachgewaschen und im Vakuum (200 mbar) getrocknet. Anschließend wird eine Probe dünnschichtchromatographisch (Dichlormethan : Methanol = 95.5) analysiert (RF= 0,62).

Ausbeute: 25,8 (87 % d. Th.)
Fp: 111 - 112°C

Beispiel 5

4-Chlor-N-[2-(2,2-difluorcyclopropyl)-ethyl]anilin

4,9 g (20 mMol) 2-(2,2-Difluorcyclopropyl)-essigsäure-4-chloranilid werden in 75 ml Tetrahydrofuran (THF) gelöst und mit 60 ml (60 mMol) einer einmolaren Lösung von Boran in THF versetzt. Nach 7 Stunden wird in wässriger Kaliumcarbonatlösung zersetzt und mit Diethylether extrahiert. Nach dem Eindampfen im Vakuum wird an der Ölpumpe (200 mbar) getrocknet und durch Säulenchromatographie (Kieselgel; Hexan : Essigester = 95 : 5) gereinigt. Eine Probe wird dünnschichtchromatographisch (Hexan : Essigester = 8 : 2) ananlysiert (RF = 0,40).

Ausbeute: 4,6 g (100 % d. Th.)
$n_D^{20}$: 1,5307

Beispiel 6

Chloressigsäure- 4-chlor-N-[2-(2,2-difluorcyclopropyl)-ethyl]-anilid

Zu einer Lösung von 4,5 g (19 mMol) N-2-(2,2-Difluorcyclopropyl)-ethyl-4-chloranilin, 2,9 ml (21 mMol) Triethylamin und 400 mg Dimethylaminopyridin in 100 ml Tetrahydrofuan (THF) tropft man bei 0°C 2,4 g (21 mMol) Chloracetylchlorid, gelöst in 5 ml THF, hinzu und rührt 2 Stunden bei Raumtemperatur nach. Der Niederschlag wird abgesaugt und die Lösung unter Vakuum (ca. 200 mbar) eingeengt. Anschließend reinigt man durch Säulenchromatographie (Kieselgel, Hexan/Essigester = 95 : 5). Eine Probe wird dünnschichtchromatographisch (Hexan : Essigster = 9 : 1) analysiert (RF = 0,14).
Ausbeute: 4,1 g (70 % d. Th.)
$n_D^{20}$: 1,5310

Beispiel 7

3,4-Dichlorphenylcarbaminsäure-[2-(2,2-difluorcyclopropyl)-ethyl]-ester

2 g (16 mMol) 2-(2,2-Difluorcyclopropyl)-ethylalkohol, gelöst in 5 ml Tetrahydrofuran (THF), werden bei 5°C mit einer Lösung von 3 g (16 mMol) 3,4-Dichlorphenylisocyanat in 5 ml THF versetzt. Nach 6 Stunden Reaktionszeit bei Raumtemperatur wird im Vakuum eingeengt und aus Cyclohexan umkristallisiert. Eine Probe wird anschließend dünnschichtchromatographisch (Hexan : Essigester = 1:1) analysiert (RF = 0,54).
Ausbeute: 3,2 g (63 % d. Th.)
Fp: 89 - 90°C

In analoger Weise werden die folgenden Verbindungen hergestellt.

Allgemeine Formel

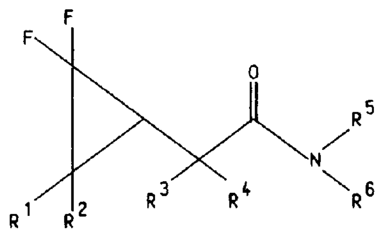

$R^1, R^2, R^3, R^4 = H$

| Beispiel Nr. | $R^5$ | $R^6$ | Physikalische Konstante $n_D^{20}$ | Fp | Kp |
|---|---|---|---|---|---|
| 8 | 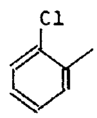 | $-C_2H_5$ | 1,5067 | | |
| 9 | 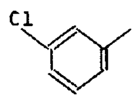 | $-C_2H_5$ | 1,5116 | | |
| 10 | 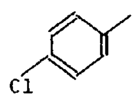 | $-C_2H_5$ | 1,5134 | | |
| 38 | 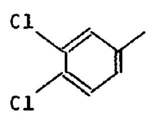 | H | | 79-80 | |
| 39 | 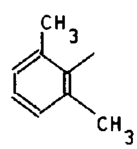 | H | | 95-96 | |
| 47 | $-(CH_2)_5-$ | | 1,4634 | | |
| 48 | $-(CH_2)_2-O-(CH_2)_2-$ | | 1,4659 | | |

| Beispiel Nr. | $R^5$ | $R^6$ | Physikalische Konstante $n_D^{20}$ | Fp | Kp |
|---|---|---|---|---|---|
| 49 | 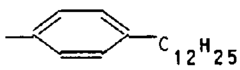 | ; H | 1,5029 | | |
| 50 | $-C_{16}H_{33}$ | ; H | | 68-69 °C | |
| 51 | $-C_{18}H_{37}$ | ; H | | 73-75 °C | |
| 52 | 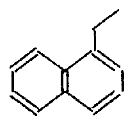 | ; H | | 100-102 °C | |
| 53 | 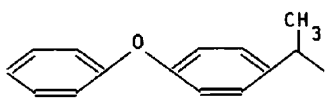 | ; H | 1,5289 | | |
| 54 | 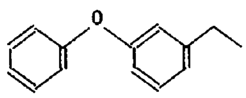 | ; H | | | |
| 55 | 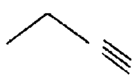 | ; H | | 56-57 °C | |
| 56 | 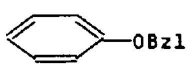 | ; H | | 157-158 °C | |
| 57 | 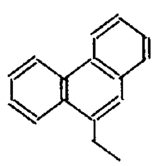 | ; H | | 172-173 °C | |
| 58 | 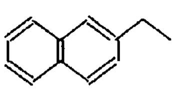 | ; H | | | |

| Beispiel Nr. | $R^5$ | $R^6$ | Physikalische Konstante $n_D^{20}$ | Fp | Kp |
|---|---|---|---|---|---|
| 59 | 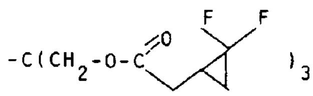 | ; H | | | |
| 60 | 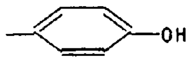 | ; H | | | |
| 61 | 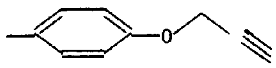 | ; H | | | |
| 62 | 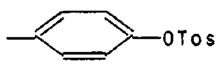 | ; H | | | |
| 63 | 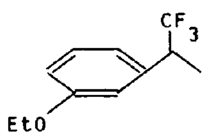 | ; H | | 85 °C | |
| 64 | 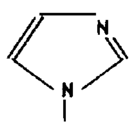 | ; H | | | |
| 65 | 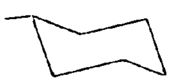 | ; 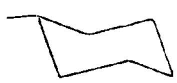 | | | |
| 66 | — H | ; H | | | |

| Beispiel Nr. | $R^5$ | | $R^6$ | Physikalische Konstante $n_D^{20}$ | Fp | Kp |
|---|---|---|---|---|---|---|
| 67 | 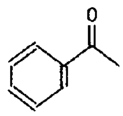 | ; | H | | | |
| 68 | 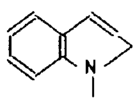 | ; | 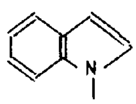 | | | |
| 69 | 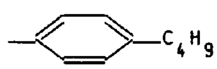 | ; | H | | 50 °C | |
| 70 | 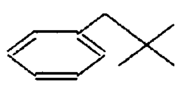 | ; | H | | 84-85 °C | |
| 71 | 1-Adamantyl | ; | H | | | |
| 72 | $-C_5H_{11}$ | ; | $-C_5H_{11}$ | | | |
| 73 | 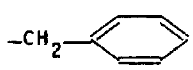 | ; | H | | 58-59 °C | |
| 74 | 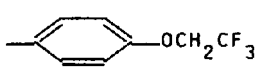 | ; | H | | | |
| 75 | 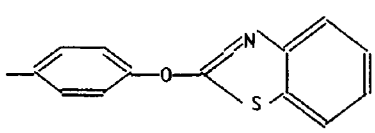 | ; | H | | | |

| Beispiel Nr. | $R^5$ | $R^6$ | Physikalische Konstante $n_D^{20}$ | Fp | Kp |
|---|---|---|---|---|---|
| 76 | 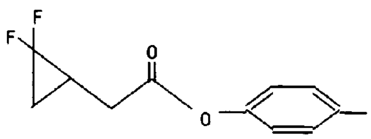 | ; H | | | |
| 90 | $-CH_2CH_2NHC=CHNO_2$ | | | 173,2 °C | |
| 108 | 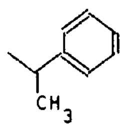 | | $R_F$ = 0,40 (Hex/EE = 1/1) | | |
| 109 | 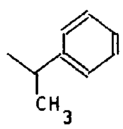 | | $R_F$ = 0,33 (Hex/EE = 1/1) | | |

Hex = Hexan
EE = Diethylethyl

Allgemeine Formel 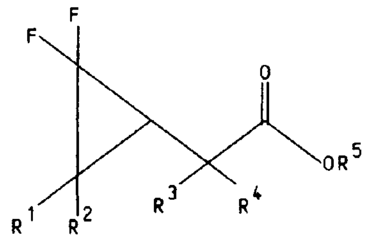 $R^1, R^2, R^3, R^4 = H$

| Beispiel Nr. | $R^5$ | Physikalische Konstante $n_D^{20}$ | Fp | Kp |
|---|---|---|---|---|
| 11 | $-C_{16}H_{33}$ | | | 131/0,02 mbar |
| 12 | 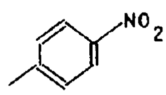 | 1,5198 | | 120/0,02 mbar |
| 13 | 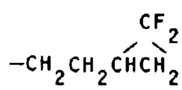 | 1,4053 | | |
| 14 | 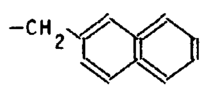 | | | 135/0,02 mbar |
| 15 | 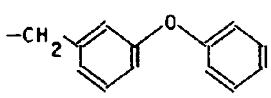 | 1,5331 | | |
| 16 | 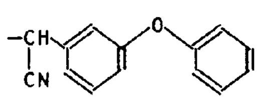 | 1,5457 | | |

| Beispiel Nr. | $R^5$ | Physikalische Konstante $n_D^{20}$ | Fp | Kp |
|---|---|---|---|---|
| 17 | 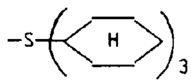 | | 93 °C | |
| 18 | 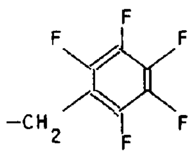 | 1,4350 | | |
| 77 | 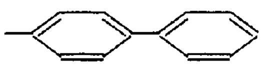 | | 73,6 °C | |
| 78 | 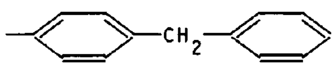 | 1,5331 | | |
| 79 | 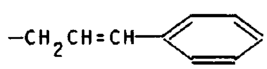 | 1,5125 | | |
| 80 | 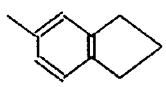 | 1,5017 | | |
| 81 | 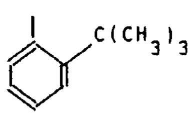 | 1,4866 | | |

| Beispiel Nr. | $R^5$ | Physikalische Konstante $n_D^{20}$ | Fp | Kp |
|---|---|---|---|---|
| 82 | 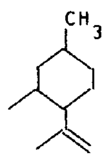 | 1,4488 | | |
| 83 | 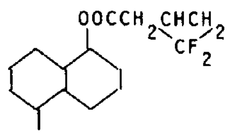 | 1,4597 | | |
| 84 | 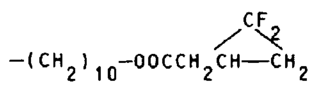 | | 34 °C | |
| 85 | 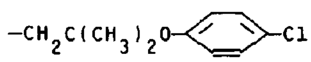 | 1,4890 | | |
| 86 | 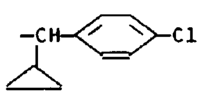 | 1,5028 | | |
| 87 | 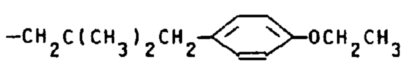 | 1,4847 | | |
| 88 | 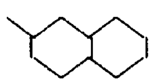 | 1,4647 | | |

| Beispiel Nr. | $R^5$ | Physikalische Konstante $n_D^{20}$ | Fp | Kp |
|---|---|---|---|---|
| 89 | 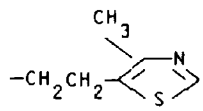 | 1,4858 | | |
| 91 | 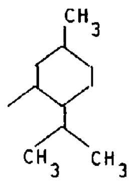 | 1,4413 | | |
| 92 | Adamantanylmethyl | 1,4776 | | |
| 93 | 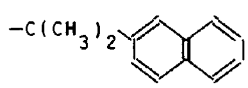 | 1,5389 | | |
| 94 | $-CH_2CH=C(CH_3)CH_2CH_2CH=C(CH_3)_2$ | 1,4549 | | |
| 95 | $-CH_2CH=C(CH_3)CH_2CH_2CH=C(CH_3)CH_2CH_2CH=C(CH_3)_2$ | 1,4686 | | |
| 96 | $-(CH_2)_6Cl$ | 1,4390 | | |
| 97 | 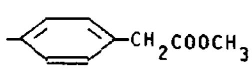 | 1,4873 | | |
| 98 | $-(CH_2)_2-C{\equiv}C-(CH_2)_4CH_3$ | 1,4396 | | |

| Beispiel Nr. | $R^5$ | Physikalische Konstante $n_D^{20}$ | Fp | Kp |
|---|---|---|---|---|
| 99 | 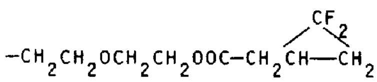 | 1,4264 | | |
| 100 | 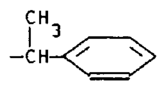 (Diastereomerengemisch) | | | |
| 101 | 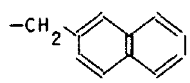 (+) | | | |
| 102 | 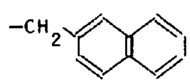 (-) | | | |
| 103 | 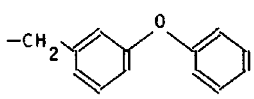 (+) | | | |
| 104 | 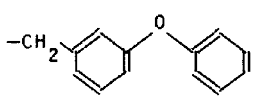 (-) | | | |
| 105 | 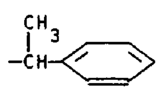 (Diastereomer 1) | | | |
| 106 | 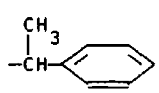 (Diastereomer 2) | | | |
| 107 | $-CH(CH_2CH_2CH_2CH_2CH_3)_2$ | 1,4249 | | |
| 110 | H (+) | | | |
| 111 | H (-) | | | |

Allgemeine Formel 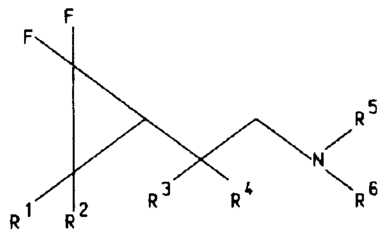 $R^1$, $R^2$, $R^3$, $R^4$ = H

| Beispiel Nr. | $R^5$ | $R^6$ | Physikalische Konstante $n_D^{20}$ | Fp | Kp |
|---|---|---|---|---|---|
| 19 | 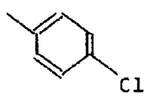 | 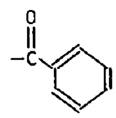 | 1,5628 | | |
| 20 | 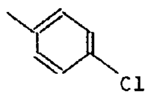 | 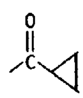 | 1,5236 | | |
| 21 | 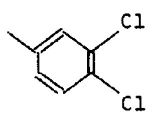 | 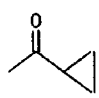 | 1,5378 | | |
| 22 | 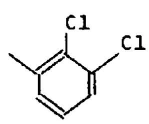 | 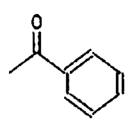 | 1,5676 | | |
| 23 | 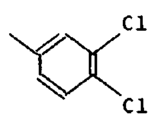 | 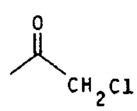 | 1,5437 | | |
| 24 | 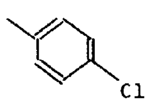 | $-CONHCH_3$ | | 92-93 °C | |
| 25 | 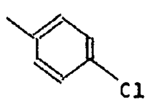 | $-COOCH_3$ | 1,5075 | | |

| Beispiel Nr. | $R^5$ | $R^6$ | Physikalische Konstante $n_D^{20}$ | Fp | Kp |
|---|---|---|---|---|---|
| 26 | 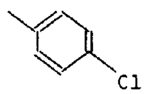 | $-COOCH(CH_3)_2$ | 1,4944 | | |
| 27 | 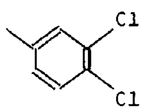 | $-CONHCH_3$ | 1,5236 | 72-73 °C | |
| 28 | 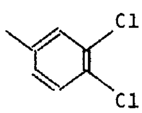 | $-COOCH_3$ | 1,5220 | | |
| 29 | 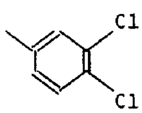 | $-COOCH(CH_3)_2$ | 1,5063 | | |
| 30 | 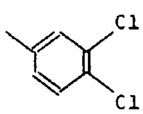 | $-COOCH_2CH_2CH_2CH_3$ | 1,5080 | | |
| 31 | 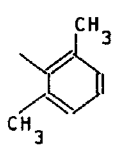 | $-COCH_2Cl$ | 1,5337 | | |
| 32 | 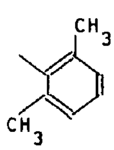 | −CO 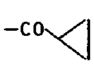 | 1,5278 | | |

| Beispiel Nr. | $R^5$ | $R^6$ | Physikalische Konstante $n_D^{20}$ | Fp | Kp |
|---|---|---|---|---|---|
| 33 | 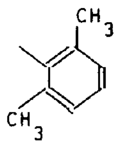 | $-COOCH_3$ | 1,5120 | | |
| 34 | 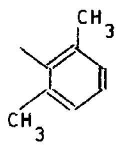 | $-COOCH_2CH_2CH_2CH_3$ | 1,5965 | | |
| 35 | 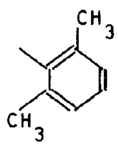 | $-COOCH_2CH(CH_3)_2$ | 1,4963 | | |
| 36 | 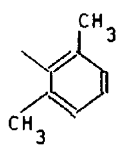 | -CO- 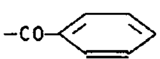 | 1,5470 | | |
| 37 | 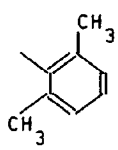 | $-CONHCH_3$ | | 80-81 °C | |
| 40 | 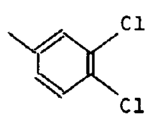 | H | 1,5439 | | |
| 41 | 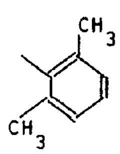 | H | 1,5014 | | |
| 42 | 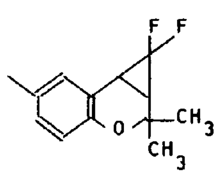 | H | 1,5110 | | |

Allgemeine Formel 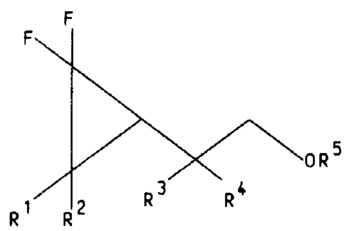 $R^1$, $R^2$, $R^3$, $R^4$ = H

| Beispiel Nr. | $R^5$ | Physikalische Konstante $n_D^{20}$ | Fp | Kp |
|---|---|---|---|---|
| 43 | 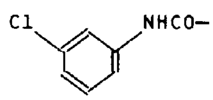 | | 54-55 °C | |
| 44 | 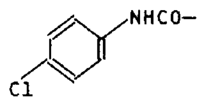 | | 64-65 °C | |
| 45 | 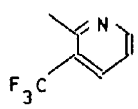 | 1,4376 | | |
| 46 | 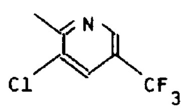 | 1,4546 | | |

Allgemeine Formel 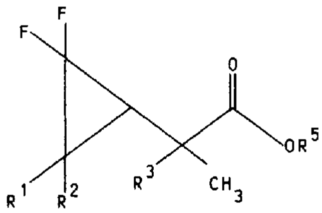 $R^1$, $R^2$, $R^3$ = H

| Beispiel Nr. | $R^5$ | | Physikalische Konstante $n_D^{20}$ | Fp | Kp |
|---|---|---|---|---|---|
| 112 | 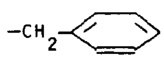 | (Diastereomerengemisch) | 1,4726 | | |
| 113 | $-C_{16}H_{33}$ | (Diastereomerengemisch) | | | |
| 114 | 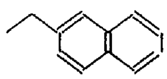 | (Diastereomer 1) | | | |
| 115 | 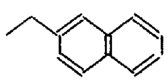 | (Diastereomer 2) | | | |
| 116 | 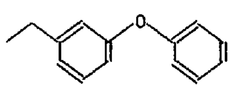 | (Diastereomer 1) | | | |
| 117 | 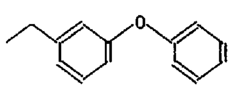 | (Diastereomer 2) | | | |
| 118 | 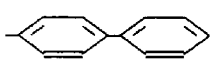 | (Diastereomer 1) | | | |
| 119 | 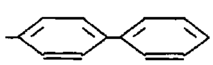 | (Diastereomer 2) | | | |

**Anwendungsbeispiel A**

Wirkung kurativer Blattbehandlung der Buschbohne (Phaseolus vulgaris nanus Aschers.) gegen bewegliche Stadien der Gemeinen Bohnenspinnmilbe (Tetranychus urticae Koch)

Im warmen Gewächshaus werden Sämlinge der Buschbohne bis zu vollständigen Entwicklung der Primärblätter angezogen und dann mit Blattstücken belegt, die von Tetranychus urticae befallen sind. Einen Tag später werden die Blattstücke entfernt und die Pflanzen mit einer 0,1 % Wirkstoff enthaltenden wäßrigen Zubereitung tropfnaß gespritzt. Nach 7 Tagen bei 22-24 °C wird der Anteil toter beweglicher Stadien von Tetranychus an den behandelten und an unbehandelten Pflanzen bestimmt. Daraus wird nach Abbott die Wirkung der Behandlung berechnet.

Eine 80-100 %ige Wirkung hatten die Verbindungen gemäß den Beispielen Nr. 1-5, 7-18, 20-22, 24, 25, 27, 28, 34, 38, 40, 43, 44 und 46.

**Anwendungsbeispiel B**

Wirkung kurativer Blattbehandlung der Buschbohne (Phaseolus vulgaris nanus Aschers.) gegen Eier der Gemeinen Bohnenspinnmilbe (Tetranychus urticae Koch)

Im warmen Gewächshaus werden Sämlinge der buschbohne bis zur vollständigen Entwicklung der Primärblätter angezogen und dann mit adulten Weibchen von Tetranychus urticae besetzt. Einen Tag später werden die Pflanzen mit den inzwischen abgelegten Eiern mit 0,1 % Wirkstoff enthaltender wäßriger Zubereitung tropfnaß gespritzt. Nach 7 Tagen bei 22-24 °C wird der Anteil abgestorbener Eier an behandelten und unbehandelten Pflanzen bestimmt. Daraus wird nach Abbott die Wirkung der Behandlung berechnet.

Eine 80-100 %ige Wirkung hatten die Verbindungen gemäß den Beispielen 1-4, 7-22, 24, 25, 27, 28, 34, 38, 40-44 und 46.

**Anwendungsbeispiel C**

Wirkung prophylaktischer Blattbehandlung gegen die Braune Reiszikade (Nilaparvata lugens Stål)

Im warmen Gewächshaus werden Reissämlinge (etwa 15 je Topf) bis zur Ausbildung des dritten Blattes angezogen und dann mit einer 0,1 % Wirkstoff enthaltenden wäßrigen Zubereitung tropfnaß gespritzt. Nach dem Antrocknen der Spritzbeläge wird über jeden Topf ein Klarsichtzylinder gestülpt. Auf jeden Topf werden dann etwa 30 Individuen der Braunen Reiszikade (Nilaparvata lugens) gebracht. Nach 2 Tagen Aufstellung bei 26 °C im Gewächshaus wird der Anteil abgetöteter Zikaden festgestellt. Unter Bezug auf einige unbehandelt gebliebene Kontrolltöpfe wird daraus nach Abbott die Wirkung berechnet.

Eine 80-100 %ige Wirkung hatten die Verbindunge gemäß den Beispielen 4, 5, 9, 17, 21, 26, 28, 29, 32, 34-36, 40 und 41.

**Anwendungsbeispiel D**

Wirkung kurativer Behandlung der Ackerbohne (Vicia faba L.) gegen die Schwarze Bohnenlaus (Aphis fabae Scop.)

Im warmen Gewächshaus werden Sämlinge der Ackerbohne (Vicia faba) , eine Pflanze je Topf, bis zu etwa 6 cm Höhe angezogen. Die Pflanzen werden dann belegt mit Zuchtmaterial der Schwarzen Bohnenlaus (Aphis fabae). Nach Besiedlung der Pflanzen mit je 100-200 Individuen werden sie mit 0,1 % Konzentration des jeweiligen Wirkstoffs in wäßriger Zubereitung tropfnaß gespritzt und im Gewächshaus bei etwa 24 °C aufgestellt. Nach 2 Tagen wird der Anteil abgetöteter Blattläuse ermittelt. Unter Bezug auf unbehandelt gebliebene Kontrolltöpfe wird nach Abbott die Wirkung berechnet.

Eine 80-100 %ige Wirkung hatten die Verbindungen gemäß den Beispielen 1, 4 und 18.

**Anwendungsbeispiel E**

Abtötende Wirkung auf Junglarven der Kohlschabe
(Plutella xylostella)

Die erfindungsgemäßen Verbindungen werden als wäßrige Emulsionen mit der Wirkstoffkonzentration 0,1 % eingesetzt. Mit diesen Wirkstoffzubereitungen werden Kohlrabiblättchen (Brassica oleracea var. gongylodes) in Polystyrol-Petrischalen dosiert (4 mg Spritzbrühe/$cm^2$) gespritzt. Nach dem Antrocknen der Spritzbeläge werden in jede Petrischale 10 Jungraupen der Kohlschabe (Plutella xylostella) eingezählt und für zwei Tage in den geschlossenen Petrischalen dem behandelten Futter exponiert. Kriterium für die Wirkungsbeurteilung ist die Sterblichkeit der Raupen in % nach 2 Tagen.
Die Verbindungen gemäß den Beispielen 17 und 28 zeigten eine 80-100 %ige Wirkung.

**Anwendungsbeispiel F**

Abtötende Wirkung auf Eier/Larven des Maiswurzelwurmes
(Diabrotica undecimpunctata)

Die erfindungsgemäßen Verbindungen werden als wäßrige Emulsionen mit der Wirkstoffkonzentration 0,1 % eingesetzt. Mit diesen Wirkstoffzubereitungen werden die Böden von Polystyrolpetrischalen sowie darin enthaltene Maiskeimlinge (1 Maiskeimling/Schale) und jeweils ca. 50 Eier des Maiswurzelwurmes (Diabrotica undecimpunctata) mit 4 mg Spritzbrühe/$cm^2$ dosiert gespritzt. Die verschlossenen Schalen werden bei 25 °C unter Langtangbedingungen für 4 Tage aufgestellt. Kriterium für die Wirkungsbeurteilung ist die Abtötung von Eiern oder der frisch schlüpfenden Larven bei Versuchsende.
Die Verbindungen gemäß den Beispielen 35, 40 und 45 zeigten eine 100 %ige Wirkung.

**Anwendungsbeispiel G**

Insektizide Wirkung auf die befruchtete weibliche Rinderzecke (Boophilus microplus, Paquera-Linie)

Gruppen mit je 5 ausgewachsenen und befruchteten weiblichen Rinderzecken werden für 10 Minuten mit einer Lösung der Testsubstanz in wäßriger Acetonlösung bei verschiedenen Konzentrationen behandelt. Anschließend werden die Zecken in Plastikbehälter eingebracht und bei 25 °C und 80 % Raumfeuchte gehalten. Die Mortalität und die Fruchtbarkeit der Zecken sowie die Lebensfähigkeit der Eier der überlebenden Zecken werden bestimmt. Die prozentuale Verringerung des gesamten Fortpflanzungsvermögens (Kombinationseffekt von Mortalität der ausgewachsenen Individuen, verminderte Fruchtbarkeit und Mortalität der Eier) wird ermittelt und mit Kontrollversuchen verglichen.

Die Kontrollversuche zeigten eine Verringerung des Fortpflanzungsvermögens von weniger als 5 %, währenddessen die Verbindungen gemäß den Beispielen 1, 3, 14, 15, 16 und 18 eine mindestens 50 %ige Verringerung des Fortpflanzungsvermögens bei einer Konzentration von 500 mg/l oder weniger ergaben.

## Patentansprüche

1. 2,2-Difluorcyclopropylethanderivate der allgemeinen Formel I

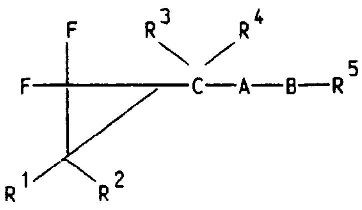

(I),

in der

$R^{1-4}$ gleich oder verschieden sein können und Wasserstoff, $C_{1-6}$-Alkyl und/oder Halogen darstellen und

$R^5$ Wasserstoff, ein Alkalimetallatom oder ein entsprechendes Äquivalent eines zweiwertigen Metalles,
$C_{1-20}$-Alkyl, $C_{2-20}$-Alkenyl, $C_{2-20}$-Alkinyl,
Halogen-$C_{1-10}$-alkyl,
$C_{3-6}$-Cycloalkyl,
$C_{1-3}$-Alkyl-$C_{3-6}$-cycloalkyl,
$C_{3-6}$-Cycloalkyl-$C_{1-6}$-alkyl,
Halogen-$C_{3-6}$-cycloalkyl-$C_{1-6}$-alkyl,
Decalinyl,
Indanyl,
Adamantyl,
Adamantylmethyl,
Difluorcyclopropylethylcarbonyloxy-$C_{1-10}$-alkyl,

Difluorcyclopropylethylcarbonyloxydecalinyl, Difluorcyclopropylcarbonyloxy-$C_{1-3}$-alkoxy-$C_{1-3}$-alkyl,
Phenyl-$C_{1-6}$-alkyl,
Phenyl-$C_{2-6}$-alkenyl,
Halogenbenzyl,
$C_{1-4}$-Alkylbenzyl,
$C_{1-3}$-Alkoxyphenyl-$C_{1-6}$-alkyl,
Phenoxybenzyl,
$\alpha$-Cyanophenoxybenzyl,
$\alpha$-$C_{1-3}$-Alkylphenoxybenzyl,
Ethoxy-($\alpha$-trifluormethyl)benzyl,
Halogenphenyl(cyclopropyl)-$C_{1-3}$-alkyl,
Halogenphenoxy-$C_{1-6}$-alkyl,
Naphthyl-$C_{1-6}$-alkyl,
Methylthiazolyl-$C_{1-6}$-alkyl,
Tris(difluorcyclopropylmethylcarbonyloxymethyl)methyl,
Tri($C_{4-8}$-cycloalkyl)stannyl, freies Aryl oder durch $C_{1-20}$-Alkyl, Halogen-$C_{1-6}$-alkyl, $C_{1-16}$-Alkoxy, Halogen-$C_{1-6}$-alkoxy, Phenyl-$C_{1-6}$-alkyl, Phenyl-$C_{1-6}$-alkoxy, $C_{3-10}$-Cycloalkyloxy, Halogen$C_{3-10}$-cycloalkyloxy, $C_{3-6}$-Cycloalkylalkoxy, Halogen-$C_{3-6}$-cycloalkylalkoxy, $C_{2-6}$-Alkenyloxy, Halogen-$C_{2-6}$alkenyloxy, $C_{2-6}$-Alkinyloxy, Halogen-$C_{2-6}$-alkinyloxy, Alkylsulfonyloxy, Alkylphenylsulfonyloxy, Halogenalkylsulfonyloxy, Phenyl, Halogen, Amino, Cyano, Hydroxy, Nitro, Aryloxy, Hetereoaryloxy, Halogenaryloxy, Arylamino, Halogenarylamino, $C_{1-6}$-Alkoxycarbonyl, $C_{1-6}$-Alkoxycarbonylmethyl, Halogen-$C_{1-6}$alkoxycarbonyl, $C_{1-2}$-Alkyldioxy, $C_{1-6}$-Alkylthio, Halogen-$C_{3-6}$-cycloalkylalkylamino, Halogen-$C_{3-6}$-cycloalkylalkylcarbonyloxy, $C_{1-6}$-Alkylamino, oder Di-$C_{1-6}$-alkylamino ein- odermehrfach substituiertes Aryl, freies Heteroaryl, durch Halogen, $C_{1-3}$-Alkyl oder Halogen-$C_{1-3}$-alkyl substituiertes Heteroaryl sowie die Gruppe $CONHR^8$ bedeutet, wobei

$R^8$ Wasserstoff, $C_{1-6}$-Alkyl, Phenyl oder durch $C_{1-6}$-Alkyl, Halogen-$C_{1-6}$-alkyl, $C_{1-16}$-Alkoxy, Halogen-$C_{1-6}$-alkoxy, Phenyl-$C_{1-6}$-alkoxy, $C_{3-10}$-Cycloalkyloxy, Halogen-$C_{3-10}$-cycloalkyloxy, $C_{3-6}$-Cycloalkylalkoxy, Halogen-$C_{3-6}$-cycloalkylalkoxy, $C_{2-6}$-Alkenyloxy, Halogen-$C_{2-6}$-alkenyloxy, $C_{2-6}$-Alkinyloxy, Halogen-$C_{2-6}$-alkinyloxy, Alkylsulfonyloxy, Halogenalkylsulfonyloxy, Phenyl, Halogen, Amino, Cyano, Hydroxy, Nitro, Aryloxy, Halogenaryloxy, Arylamino, Halogenarylamino, $C_{1-6}$-Alkoxycarbonyl, Halogen-$C_{1-6}$-alkoxycarbonyl, $C_{1-2}$-Alkyldioxy, $C_{1-6}$-Alkylthio, Halogen-$C_{3-6}$-cycloalkylalkylamino, $C_{1-6}$-Alkylamino, oder Di-$C_{1-6}$-alkylamino ein- oder mehrfach substituiertes Phenyl bedeutet,

A für eine Carbonyl, Thiocarbonyl oder Methylen-Gruppe und
B für Sauerstoff, Schwefel oder die Gruppe $NR^6$ steht, in der
$R^6$ Wasserstoff oder $C_{1-6}$-Alkyl, $C_{3-12}$-Cycloalkyl, Phenyl sowie die Gruppe $CO-R^7$ bedeutet, wobei
$R^7$ $C_{1-6}$-Alkyl, Halogen-$C_{1-6}$-alkyl, Phenyl oder durch $C_{1-6}$-Alkyl, Halogen-$C_{1-6}$-alkyl, $C_{1-16}$-Alkoxy, Halogen-$C_{1-6}$-alkoxy, Phenyl-$C_{1-6}$-alkoxy, $C_{3-10}$-Cycloalkyloxy, Halogen-$C_{3-10}$-cycloalkyloxy, $C_{3-6}$-Cycloalkylalkoxy, Halogen-$C_{3-6}$-cycloalkylalkoxy, $C_{2-6}$-Alkenyloxy, Halogen-$C_{2-6}$-alkenyloxy, $C_{2-6}$-Alkinyloxy, Halogen-$C_{2-6}$-alkinyloxy, Alkylsulfonyloxy, Halogenalkylsulfonyloxy, Phenyl, Halogen, Amino, Cyano, Hydroxy, Nitro, Aryloxy, Halogenaryloxy, Arylamino, Halogenarylamino, $C_{1-6}$-Alkoxycarbonyl, Halogen-$C_{1-6}$-alkoxycarbonyl, $C_{1-2}$-Alkyldioxy, $C_{1-6}$-Alkylthio, Halogen-$C_{3-6}$-cycloalkylalkylamino, $C_{1-6}$-Alkylamino, oder Di-$C_{1-6}$alkylamino ein- oder mehrfach substituiertes Phenyl, $C_{3-6}$-Cycloalkyl, $C_{1-6}$ Alkylamino oder $C_{1-6}$-Alkoxy bedeutet.

2. 2,2-Difluorcyclopropylethanderivate nach Anspruch 1 der Formel I, worin
A eine Carbonylgruppe,
B Sauerstoff oder Gruppe NH,
$R^{1-4}$ Wasserstoff und
$R^5$ Wasserstoff, $C_{1-20}$-Alkyl, $C_{2-20}$-Alkenyl, $C_{2-20}$-Alkinyl, m-Phenoxybenzyl und Naphthylmethyl
bedeuten.

3. Verbindungen der allgemeinen Formel II

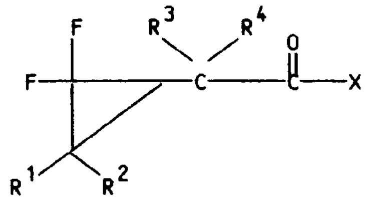

(II),

worin

$R^{1-4}$ die in Formel I angegebene Bedeutung haben und

X für OH, Cl oder Br steht, als Zwischenprodukte zur Herstellung von Verbindungen der Formel I.

4. Verfahren zur Herstellung von 2,2-Difluorcyclopropylethanderivaten der Formel I, dadurch gekennzeichnet, daß man falls A für die Carbonylgruppe steht

A) ein Säurehalogenid der allgemeinen Formel II

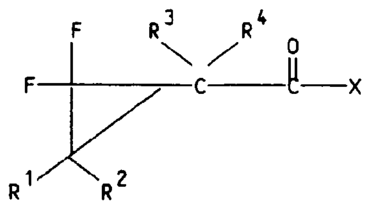

(II),

worin

$R^{1-4}$ die in Formel I angegebene Bedeutung haben und

X für Chlor oder Brom steht, mit einem Alkohol oder Amin der allgemeinen Formel III

$$H\text{-}B\text{-}R^5 \qquad (III),$$

worin

B und $R^5$ die in Formel I angegebene Bedeutung haben, gegebenenfalls in einem Lösungsmittel in Gegenwart eines Säureacceptors umsetzt, oder

B) eine freie Säure der allgemeinen Formel IV

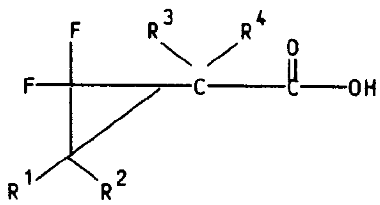

(IV),

worin

$R^{1-4}$ die in Formel I angegebene Bedeutung haben, mit einem Alkohol oder Amin der Formel III, gegebenenfalls unter Verwendung eines Lösungsmittels in Gegenwart eines Katalysators, umsetzt, oder

C) eine Säure der allgemeinen Formel V

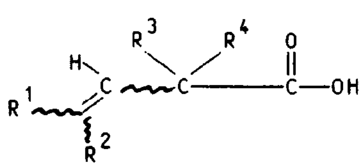

(V),

worin

$R^{1-4}$ die in Formel I angegebene Bedeutung haben, mit einem Alkohol oder Amin der Formel III, gegebenenfalls in einem Lösungsmittel in Gegenwart Katalysators oder wasserentziehender Mittel zu einer

Zwischenverbindung der Formel VI

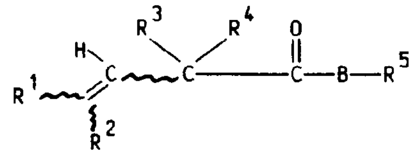

(VI),

worin

B und $R^{1-5}$ die in Formel I angegebene Bedeutung haben, umsetzt und diese in Gegenwart eines inerten Lösungsmittels mit Difluorcarben reagieren läßt, oder falls A eine Methylengruppe bedeutet und B die Gruppe $NR^6$ ist,

D) ein Amid der speziellen Formel I

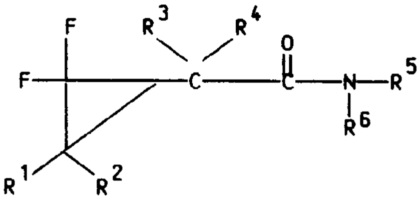

(I),

worin

$R^{1-6}$ die in Formel I angegebene Bedeutung haben, gegebenenfalls in einem Lösungsmittel in Gegenwart eines Katalysators, mit einem Reduktionsmittel umsetzt, oder

E) ein Amin der allgemeinen Formel VII

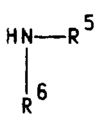

(VII),

worin

$R^5$ und $R^6$ die in Formel I angegebene Bedeutung haben, in Gegenwart einer Base, mit einem Halogenid der Formel VIII

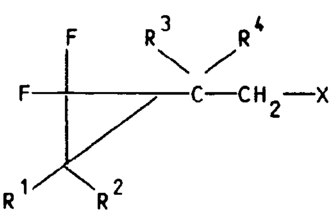

(VIII),

worin

$R^{1-4}$ die in Formel I angegebene Bedeutung haben und X für Chlor oder Brom steht, gegebenenfalls unter Verwendung eines Lösungsmittels, umsetzt, oder

F) Verbindungen der allgemeinen Formel IX

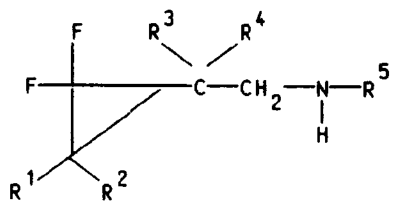

(IX),

worin

$R^{1-5}$ die in Formel I angegebene Bedeutung haben und nach der Reaktionsvariante D) oder E) hergestellt werden, mit Säurehalogeniden der allgemeinen Formel X

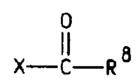

(X),

worin

$R^8$ die in Formel I angegebene Bedeutung hat und X für Chlor oder Brom steht, gegebenenfalls unter Verwendung eines Lösungsmittels in Gegenwart eines Säureacceptors, umsetzt, oder falls B Sauerstoff ist,

G) einen Alkohol der allgemeinen Formel XI

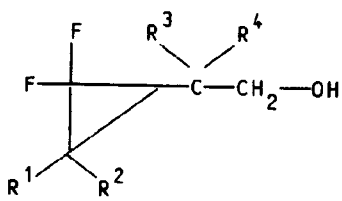

(XI),

worin

$R^{1-4}$ die in Formel I angegebene Bedeutung haben, mit einem Isocyanat der allgemeinen Formel XII

$$O=C=N-R^8 \qquad (XII),$$

worin

$R^8$ die in Formel I angegebene Bedeutung hat, gegebenenfalls unter Verwendung eines Lösungsmittels in Gegenwart eines Katalysators, umsetzt, und gegebenenfalls anschließend die so erhaltenen racemischen Gemische in die optisch aktiven Isomeren nach an sich bekannten Methoden auftrennt.

5. Schädlingsbekämpfungsmittel, gekennzeichnet durch einen Gehalt an mindestens einer Verbindung gemäß den Ansprüchen 1 und 2 der Formel I.

6. Schädlingsbekämpfungsmittel gemäß Anspruch 5 in Mischung mit Träger- und/oder Hilfsstoffen.

7. Verwendung von Verbindungen gemäß den Ansprüchen 1 und 2 der Formel I zur Bekämpfung von Insekten und Milben.

## Claims

1. 2.2-Difluorocyclopropylethane derivatives of general formula I

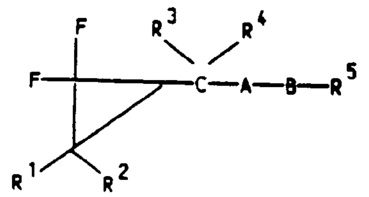

(I),

in which

$R^{1-4}$ are the same or different and are hydrogen, $C_{1-6}$-alkyl and/ or halogen,

$R^5$ is hydrogen, an alkali metal or a corresponding equivalent of a divalent metal,
$C_{1-20}$-alkyl, $C_{2-20}$-alkenyl, $C_{2-20}$-alkynyl,
halo-$C_{1-10}$-alkyl,
$C_{3-6}$-cycloalkyl,
$C_{1-3}$-alkyl-$C_{3-6}$-cycloalkyl,
$C_{3-6}$-cycloalkyl-$C_{1-6}$-alkyl,
halo-$C_{3-6}$-cycloalkyl-$C_{1-6}$-alkyl,
decalinyl,
indanyl,
adamantyl,
adamantylmethyl,
difluorocyclopropylethylcarbonyloxy-$C_{1-10}$-alkyl,
difluorocyclopropylethylcarbonyloxydecalinyl, difluorocyclopropylcarbonyloxy-$C_{1-3}$-alkoxy-$C_{1-3}$-alkyl,
phenyl-$C_{1-6}$-alkyl,
phenyl-$C_{2-6}$-alkenyl,
halobenzyl,
$C_{1-4}$-alkylbenzyl,
$C_{1-3}$-alkoxyphenyl-$C_{1-6}$-alkyl,
phenoxybenzyl,
$\alpha$-cyanophenoxybenzyl,
$\alpha$-$C_{1-3}$-alkylphenoxybenzyl,
ethoxy-($\alpha$-trifluoromethyl)benzyl,
halophenyl(cyclopropyl)-$C_{1-3}$-alkyl,
halophenoxy-$C_{1-6}$-alkyl,
naphthyl-$C_{1-6}$-alkyl,
methylthiazolyl-$C_{1-6}$-alkyl,
tris(difluorocyclopropylmethylcarbonyloxymethyl)methyl,
tri($C_{4-8}$-cycloalkyl)stannyl, aryl, optionally substituted by one or more of $C_{1-20}$-alkyl, halo-$C_{1-6}$-alkyl, $C_{1-16}$-alkoxy, halo-$C_{1-6}$-alkoxy, phenyl-$C_{1-6}$-alkyl, phenyl-$C_{1-6}$-alkoxy, $C_{3-10}$-cycloalkoxy, halo-$C_{3-10}$-cycloalkoxy, $C_{3-6}$-cycloalkylalkoxy, halo-$C_{3-6}$-cycloalkylalkoxy, $C_{2-6}$-alkenyloxy, halo-$C_{2-6}$-alkenyloxy, $C_{2-6}$-alkynyloxy, halo-$C_{2-6}$-alkynyloxy, alkylsulphonyloxy, alkylphenylsulphonyloxy, haloalkylsulphonyloxy, phenyl, halogen, amino, cyano, hydroxy, nitro, aryloxy, heteroaryloxy, haloaryloxy, arylamino, haloarylamino, $C_{1-6}$-alkoxycarbonyl, $C_{1-6}$-alkoxycarbonylmethyl, halo-$C_{1-6}$-alkoxycarbonyl, $C_{1-2}$-alkylenyldioxy, $C_{1-6}$-alkylthio, halo-$C_{3-6}$-cycloalkylalkylamino, halo-$C_{3-6}$-cycloalkylalkylcarbonyloxy, $C_{1-6}$-alkylamino or di-$C_{1-6}$-alkylamino, heteroaryl, optionally substituted by halogen, $C_{1-3}$-alkyl or halo-$C_{1-3}$-alkyl, or the group $CONHR^8$, in which

$R^8$ is hydrogen, $C_{1-6}$-alkyl or phenyl, optionally substituted by one or more of $C_{1-6}$-alkyl, halo-$C_{1-6}$-alkyl, $C_{1-16}$-alkoxy, halo-$C_{1-6}$-alkoxy, phenyl-$C_{1-6}$-alkoxy, $C_{3-10}$-cycloalkoxy, halo-$C_{3-10}$-cycloalkoxy, $C_{3-6}$-cycloalkylalkoxy, halo-$C_{3-6}$-cycloalkylalkoxy, $C_{2-6}$-alkenyloxy, halo-$C_{2-6}$-alkenyloxy, $C_{2-6}$-alkynyloxy, halo-$C_{2-6}$-alkynyloxy, alkyl-sulphonyloxy, haloalkylsulphonyloxy, phenyl, halogen, amino, cyano, hydroxy, nitro, aryloxy, haloaryloxy, arylamino, haloarylamino, $C_{1-6}$-alkoxycarbonyl, halo-$C_{1-6}$-alkoxycarbonyl, $C_{1-2}$-alkyldioxy, $C_{1-6}$-alkylthio, halo-$C_{3-6}$-cycloalkylalkylamino, $C_{1-6}$-alkylamino or di-$C_{1-6}$-alkylamino,

A is carbonyl, thiocarbonyl or methylene,

B is oxygen, sulphur or the group $NR^6$, in which

$R^6$ is hydrogen, $C_{1-6}$-alkyl or $C_{3-12}$-cycloalkyl, phenyl or the group $COR^7$, in which

$R^7$ is $C_{1-6}$-alkyl; halo-$C_{1-6}$-alkyl; phenyl, optionally substituted by one or more of $C_{1-6}$-alkyl, halo-$C_{1-6}$-alkyl,

$C_{1-16}$-alkoxy, halo-$C_{1-6}$-alkoxy, phenyl-$C_{1-6}$-alkoxy, $C_{3-10}$-cycloalkoxy, halo-$C_{3-10}$-cycloalkoxy, $C_{3-6}$-cycloalkylalkoxy, halo-$C_{3-6}$-cycloalkylalkoxy, $C_{2-6}$-alkenyloxy, halo-$C_{2-6}$-alkenyloxy, $C_{2-6}$-alkynyloxy, halo-$C_{2-6}$-alkinyloxy, alkylsulphonyloxy, haloalkylsulphonyloxy, phenyl, halogen, amin, cyano, hydroxy, nitro, aryloxy, haloaryloxy, arylamino, haloarylamino, $C_{1-6}$-alkoxycarbonyl, halo-$C_{1-6}$-alkoxycarbonyl, $C_{1-2}$-alkylenedioxy, $C_{1-6}$-alkylthio, halo-$C_{3-6}$-cycloalkylalkylamino, $C_{1-6}$-alkylamino or di-$C_{1-6}$-alkylamino; $C_{3-6}$-cycloalkyl, $C_{1-6}$-alkylamino or $C_{1-6}$-alkoxy.

2. 2.2-Difluorocyclopropylethane derivatives according to claim 1 of formula I, in which
A is a carbonyl group,
B is oxygen or the group NH,
$R^{1-4}$ are hydrogen and
$R^5$ is hydrogen, $C_{1-20}$-alkyl, $C_{2-20}$-alkenyl, $C_{2-20}$-alkynyl m-phenoxybenzyl or naphthylmethyl.

3. Compounds of general formual II

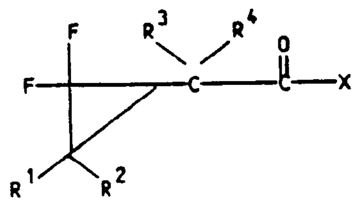
(II),

as intermediates in the production of compounds of formula I, in which
$R^{1-4}$ have the meanings given in formula I and
X is OH, Cl or Br.

4. Process for the preparation of 2,2-difluorocyclopropylethane derivatives of the general formula I, characterised by, when A is the carbonyl group,

A) reacting an acid halide of general formula II

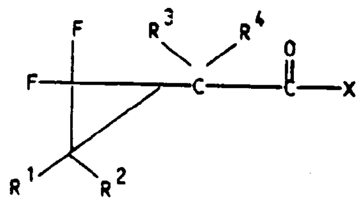
(II),

in which
$R^{1-4}$ have the meanings given in formula I and
X is chloro or bromo, with an alcohol or amine of general formula III

$$H\text{-}B\text{-}R^5 \qquad (III),$$

in which
B and $R^5$ have the meanings given in formula I, optionally in a solvent and in the presence of an acid acceptor, or

B) reacting a free acid of general formula IV

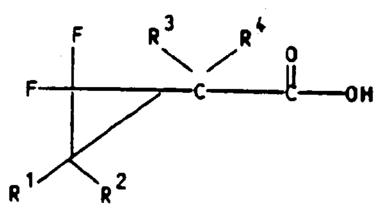
(IV),

in which

$R^{1-4}$ have the meanings given in formula I, with an alcohol or amine of formula III, optionally using a solvent in the presence of a catalyst, or

C) reacting an acid of general formula V

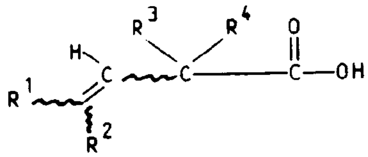

(V),

in which

$R^{1-4}$ have the meanings given in formula I, with an alcohol or amine of formula III, optionally in a solvent in the presence of a catalyst or a dehydrating agent, to give an intermediate of formula VI

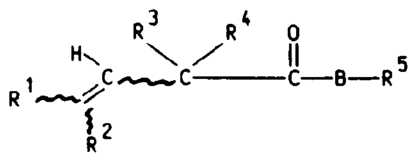

(VI),

in which

B and $R^{1-5}$ have the meanings given in formula I, and reacting this with difluorocarbene, in the presence of an inert solvent, or when A is methylene and B is the group $NR^6$, the compounds can be prepared,

D) by reacting an amide of the specific formula I

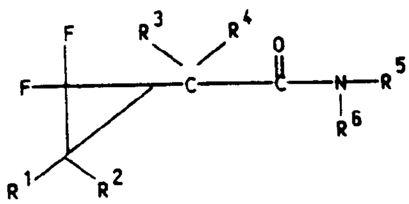

(I),

in which

$R^{1-6}$ have the meanings given in formula I, with a reducing agent, optionally in a solvent and in the presence of a catalyst, or

E) by reacting an amine of general formula VII

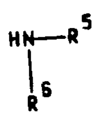

(VII),

in which

$R^5$ and $R^6$ have the meanings given in formula I, in the presence of a base, with a halide of general formula VIII

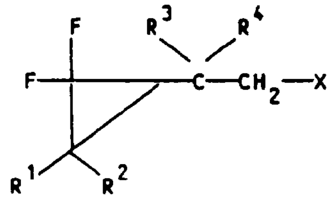

(VIII),

in which

$R^{1-4}$ have the meanings given in formula I and X is chlorine or bromine, optionally using a solvent, or

F) by reacting compounds of general formula IX

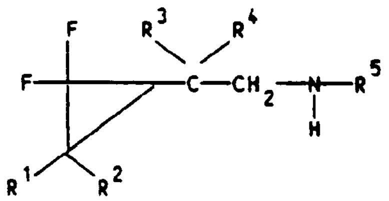

(IX),

in which

$R^{1-5}$ have the meanings given in formula I, and prepared according to reaction variants D) or E), with acid halides of general formula X

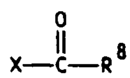

(X),

in which

$R^8$ has the meaning given in formula I and X is chlorine or bromine, optionally using a solvent in the presence of an acid acceptor, or when B is oxygen, the compounds can be prepared,

G) by reacting an alcohol of general formula XI

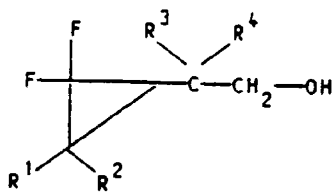

(XI),

in which

$R^{1-4}$ have the meanings given in formula I, with an isocyanate of general formula XII

$$O = C = N - R^8 \qquad \text{(XII)},$$

in which

$R^8$ has the meaning given in formula I, optionally using a solvent in the presence of a catalyst and optionally, subsequently separating the racemic mixture into optically active isomers according to known methods.

5. A pesticidal composition characterised in that it comprises at least a compound as claimed in claim 1 and 2 of formula I.

6. A pesticidal composition according to claim 5, in admixture with diluents and/or carriers.

7. Use of compounds according to claim 1 and 2 of formula I, for combating insects and acarids.

## Revendications

1. (Difluoro-2,2 cyclopropyl)-éthanes répondant à la formule générale I :

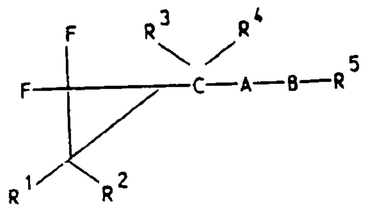

(I),

dans laquelle

$R^1$ à $R^4$ peuvent être identiques ou différents et représentent chacun l'hydrogène, un alkyle en $C_1$-$C_6$ et/ou un halogène,

$R^5$ représente l'hydrogène, un atome de métal alcalin, un équivalent correspondant d'un métal divalent, un alkyle en $C_1$-$C_{20}$, un alcényle en $C_2$-$C_{20}$, un alcynyle en $C_2$-$C_{20}$,
un halogéno-$C_1$-$C_{10}$-alkyle,
un $C_3$-$C_6$-cycloalkyle,
un $C_1$-$C_3$-alkyl-$C_3$-$C_6$-cycloalkyle,
un $C_3$-$C_6$-cycloalkyl-$C_1$-$C_6$-alkyle,
un halogéno-$C_3$-$C_6$-cycloalkyl-$C_1$-$C_6$-alkyle,
un décalinyle,
un indanyle,
un adamantyle,
un adamantyl-méthyle,
un (difluorocyclopropyl)-éthylcarbonyloxy-$C_1$-$C_{10}$-alkyle,
un (difluorocyclopropyl)-éthylcarbonyloxy-décalinyle, un (difluorocyclopropyl)-carbonyloxy-$C_1$-$C_3$-alcoxy-$C_1$-$C_3$-alkyle,
un phényl-$C_1$-$C_6$-alkyle,
un phényl-$C_2$-$C_6$-alcényle,
un halogénobenzyle,
un $C_1$-$C_4$-alkylbenzyle,
un $C_1$-$C_3$-alcoxyphényl-$C_1$-$C_6$-alkyle,
un phénoxybenzyle,
un $\alpha$-cyanophénoxybenzyle,
un $\alpha$-$C_1$-$C_3$-alkylphénoxybenzyle,
un éthoxy-($\alpha$-trifluorométhyl)-benzyle,
un halogénophényl-(cyclopropyl)-$C_1$-$C_3$-alkyle,
un halogénophénoxy-$C_1$-$C_6$-alkyle,
un naphtyl-$C_1$-$C_6$-alkyle,
un méthyl-thiazolyl-$C_1$-$C_6$-alkyle,
un tris-(difluorocyclopropyl-méthyl-carbonyloxyméthyl)-méthyle,
un tris-($C_4$-$C_8$-cycloalkyl)-stannyle, un aryle libre, un aryle porteur d'un ou de plusieurs substituants pris dans l'ensemble constitué par les radicaux $C_1$-$C_{20}$-alkyles, halogéno-$C_1$-$C_6$-alkyles, $C_1$-$C_{16}$-alcoxy, halogéno-$C_1$-$C_6$-alcoxy, phényl-$C_1$-$C_6$-alkyles, phényl-$C_1$-$C_6$-alcoxy, $C_3$-$C_{10}$-cycloalkyloxy, halogéno-$C_3$-$C_{10}$-cycloalkyloxy, $C_3$-$C_6$-cycloalkyl-alcoxy, halogéno-$C_3$-$C_6$-cycloalkyl-alcoxy, $C_2$-$C_6$-alcényloxy, halogéno-$C_2$-$C_6$-alcényloxy, $C_2$-$C_6$-alcynyloxy, halogéno-$C_2$-$C_6$-alcynyloxy, arylsulfonyloxy, alkylphénylsulfonyloxy, halogénoalkylsulfonyloxy, phényles, halogéno, amino, cyano, hydroxy, nitro, aryloxy, hétéro-aryloxy, halogéno-aryloxy, arylamino, halogéno-arylamino, $C_1$-$C_6$-alcoxycarbonyles, $C_1$-$C_6$-alcoxycarbonylméthyles, halogéno-$C_1$-$C_6$-alcoxycarbonyles, $C_1$-$C_2$-alkyl-dioxy, $C_1$-$C_6$-alkylthio, halogéno-$C_3$-$C_6$-cycloalkyl-alkylamino, halogéno-$C_3$-$C_6$-cycloalkyl-alkylcarbonyloxy, $C_1$-$C_6$-alkylamino et di-$C_1$-$C_6$-alkylamino, un hétéro-aryle libre, un hétéro-aryle porteur d'un halogène, d'un alkyle en $C_1$-$C_3$ ou d'un halogéno-alkyle en $C_1$-$C_3$, ou encore un radical -CONHR$^8$ dans lequel

$R^8$ représente l'hydrogène, un alkyle en $C_1$-$C_6$, un phényle ou un phényle porteur d'un ou de plusieurs substituants pris dans l'ensemble constitué par les radicaux alkyles en $C_1$-$C_6$, halogéno-$C_1$-$C_6$-alkyles, $C_1$-$C_{16}$-alcoxy, halogéno-$C_1$-$C_6$-alcoxy, phényl-$C_1$-$C_6$-alcoxy, $C_3$-$C_{10}$-cycloalkyloxy, halogéno-$C_3$-$C_{10}$-cycloalkyloxy, $C_3$-$C_6$-cycloalkyl-alcoxy, halogéno-$C_3$-$C_6$-cycloalkyl-alcoxy, $C_2$-$C_6$-alcényloxy, halogéno-$C_2$-$C_6$-alcényloxy, $C_2$-$C_6$-alcynyloxy, halogéno-$C_2$-$C_6$-alcynyloxy, alkylsulfonyloxy, halogénoalkylsulfonyloxy, phényles, halogéno, amino, cyano, hydroxy, nitro, aryloxy, halogéno-aryloxy, arylamino, halogénoarylamino, $C_1$-$C_6$-alcoxycarbonyles, halogéno-$C_1$-$C_6$-alcoxycarbonyles, $C_1$-$C_2$-alkyl-dioxy, $C_1$-$C_6$-alkylthio, halogéno-$C_3$-$C_6$-cycloalkyl-alkylamino, $C_1$-$C_6$-alkylamino et di-$C_1$-$C_6$-alkylamino,

A représente un radical carbonyle, carbothioyle ou méthylène et

B représente l'oxygène, le soufre ou un radical $NR^6$ dans lequel

$R^6$ représente l'hydrogène, un alkyle en $C_1$-$C_6$, un cycloalkyle en $C_3$-$C_{12}$, un phényle ou un radical -CO-$R^7$ dans lequel

$R^7$ représente un $C_1$-$C_6$-alkyle, un halogéno-$C_1$-$C_6$-alkyle, un phényle, un phényle porteur d'un ou de plusieurs substituants pris dans l'ensemble constitué par les radicaux $C_1$-$C_6$-alkyles, halogéno-$C_1$-$C_6$-alkyles, $C_1$-$C_{16}$-alcoxy, halogéno-$C_1$-$C_6$-alcoxy, phényl-$C_1$-$C_6$-alcoxy, $C_3$-$C_{10}$-cycloalkyloxy, halogéno-$C_3$-$C_{10}$-cycloalkyloxy, $C_3$-$C_6$-cycloalkyl-alcoxy, halogéno-$C_3$-$C_6$-cycloalkyl-alcoxy, $C_2$-$C_6$-alcényloxy, halogéno-$C_2$-$C_6$-alcényloxy, $C_2$-$C_6$-alcynyloxy, halogéno-$C_2$-$C_6$-alcynyloxy, alkylsulfonyloxy, halogénoalkylsulfonyloxy, phényles, halogéno, amino, cyano, hydroxy, nitro, aryloxy, halogéno-aryloxy, arylamino, halogénoarylamino, $C_1$-$C_6$-alcoxycarbonyles, halogéno-$C_1$-$C_6$-alcoxycarbonyles, $C_1$-$C_2$-alkyl-dioxy, $C_1$-$C_6$-alkylthio, halogéno-$C_3$-$C_6$-cycloalkyl-alkylamino, $C_1$-$C_6$-alkylamino et di-$C_1$-$C_6$-alkylamino, un $C_3$-$C_6$-cycloalkyle, un $C_1$-$C_6$-alkylamino ou un $C_1$-$C_6$-alcoxy.

2. (Difluoro-2,2 cyclopropyl)-éthanes de formule I selon la revendication 1, dans lesquels

A représente un radical carbonyle,

B représente l'oxygène ou un radical NH,

$R^1$ à $R^4$ représentent chacun l'hydrogène et

$R^5$ représente l'hydrogène, un alkyle en $C_1$-$C_{20}$, un alcényle en $C_2$-$C_{20}$, un alcynyle en $C_2$-$C_{20}$, un m-phénoxy-benzyle ou un naphtylméthyle.

3. Composés répondant à la formule générale II :

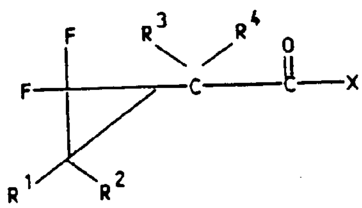

(II),

dans laquelle

$R^1$ à $R^4$ ont les significations qui leur ont données à propos de la formule I et

X représente OH, Cl ou Br, en tant que corps intermédiaires pour la préparation de composés de formule I.

4. Procédé pour préparer des (difluoro-2,2 cyclopropyl)-éthanes de formule I, procédé caractérisé en que, dans le cas où A représente un radical carbonyle :

A) on fait réagir un halogénure d'acide répondant à la formule générale II :

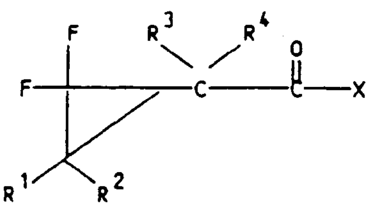

(II),

dans laquelle

$R^1$ à $R^4$ ont les significations qui leur ont données à propos de la formule I et

X représente le chlore ou le brome, avec un alcool ou une amine répondant à la formule générale III :

$$H\text{-}B\text{-}R^5 \qquad (III),$$

dans laquelle

B et $R^5$ ont les significations qui leur ont données à la revendication 1, éventuellement dans un solvant, en présence d'un accepteur d'acides, ou

B) on fait réagir un acide libre répondant à la formule générale IV :

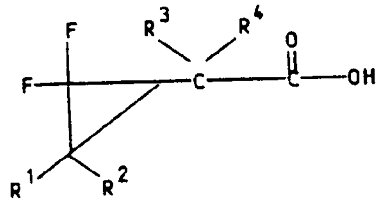

(IV),

dans laquelle

$R^1$ à $R^4$ ont les significations qui leur ont données à propos de la formule I, avec un alcool ou une amine de formule III, éventuellement en utilisant un solvant, en présence d'un catalyseur, ou

C) on fait réagir un acide répondant à la formule générale V :

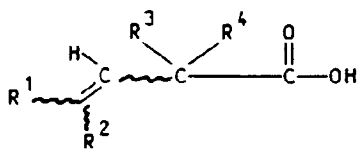

(V),

dans laquelle

$R^1$ à $R^4$ ont les significations qui leur ont données à propos de la formule I, avec un alcool ou une amine de formule III, éventuellement en utilisant un solvant, en présence d'un catalyseur ou d'un agent déshydratant, de manière à obtenir un corps intermédiaire répondant à la formule VI :

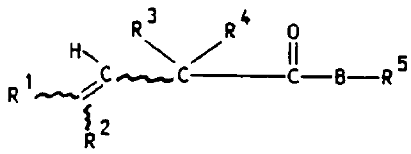

(VI),

dans laquelle

B et $R^1$ à $R^5$ ont les significations qui leur ont données à propos de la formule I, et on fait réagir celui-ci, en présence d'un solvant inerte, avec le difluorocarbène, ou, dans le cas où A représente un radical méthylène et B un radical $NR^6$ :

D) on fait réagir un amide répondant à la formule I particulière suivante :

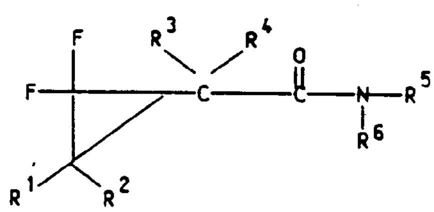

(I),

dans laquelle

$R^1$ à $R^6$ ont les significations qui leur ont été données à propos de la formule I, éventuellement dans un solvant, en présence d'un catalyseur, avec un agent de réduction, ou

E) on fait réagir une amine répondant à la formule générale VII :

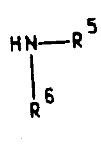

(VII),

dans laquelle

$R^5$ et $R^6$ ont les significations qui leur ont été données à propos de la formule I, en présence d'une

base, avec un halogénure répondant à la formule VIII :

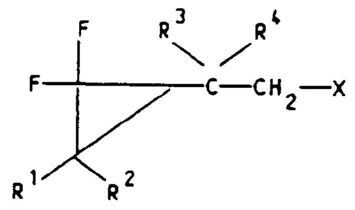

(VIII),

dans laquelle

$R^1$ à $R^4$ ont les significations qui leur ont été données à propos de la formule I et X représente le chlore ou le brome, éventuellement en utilisant un solvant, ou

F) on fait réagir des composés répondant à la formule générale IX :

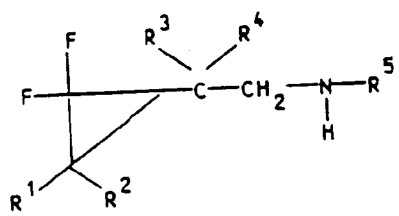

(IX),

dans laquelle

$R^1$ et $R^5$ ont les significations qui leur ont été données à propos de la formule I, composés qui ont été préparés selon l'une des variantes D) et E), avec des halogénures d'acides répondant à la formule générale X :

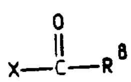

(X),

dans laquelle

$R^8$ a la signification qui lui a été donnée à propos de la formule I et X représente le chlore ou le brome, éventuellement en utilisant un solvant, en présence d'un accepteur d'acides, ou, dans le cas où B représente l'oxygène :

G) on fait réagir un alcool répondant à la formule générale XI :

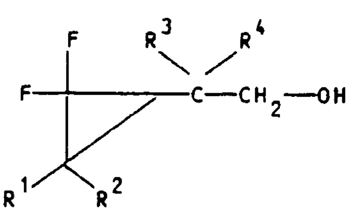

(XI),

dans laquelle

$R^1$ à $R^4$ ont les significations qui leur ont été données à propos de la formule I, avec un isocyanate répondant à la formule générale XII :

$$O=C=N\text{-}R^8 \qquad (XII),$$

dans laquelle

$R^8$ a la signification qui lui a été donnée à propos de la formule I, éventuellement en utilisant un solvant, en présence d'un catalyseur, et ensuite, le cas échéant, on sépare les mélanges racémiques ainsi obtenus en les isomères optiquement actifs, par des méthodes connues.

5. Produits pesticides caractérisés en ce qu'ils contiennent au moins un composé de formule I selon l'une des revendications 1 et 2.

6. Produits pesticides selon la revendication 5 en mélange avec des supports et/ou des adjuvants.

7. Application de composés de formule I selon l'une des revendications 1 et 2 à la lutte contre des insectes et des acariens.